# FASCICULE DE BREVET EUROPEEN

(11) **EP 3 142 779 B1**
(45) Date de publication et mention de la délivrance du brevet: **26.04.2023**
(21) Numéro de dépôt: 15724206.6
(22) Date de dépôt: 13.05.2015
(51) Int. Cl.: B05B 7/00, B01F 23/20

(54) **INSTALLATION ET PROCÉDÉ DE PRODUCTION DE MOUSSE**
SCHAUMSTOFFHERSTELLUNGSAUSRÜSTUNG UND SCHAUMSTOFFHERSTELLUNGSVERFAHREN
FOAM PRODUCTION EQUIPMENT AND METHOD

(30) Priorité: 16.05.2014 FR 1454412
(43) Date de publication de la demande: 22.03.2017
(73) Titulaire: Poulnais, Eric, 78150 Le Chesnay (FR)
(72) Inventeur: Poulnais, Eric, 78150 Le Chesnay (FR)
(74) Mandataire: Vidon Brevets & Stratégie
(86) Numéro de dépôt international: PCT/EP2015/060685
(87) Numéro de publication internationale: WO 2015/173350

(56) Documents cités:
- EP-A1- 0 407 347
- WO-A1-2009/109354
- WO-A2-03/006586
- CH-A- 542 914
- GB-A- 1 355 283
- GB-A- 2 202 503
- JP-A- S61 212 318
- US-A- 4 732 181
- US-A- 4 884 076
- US-B1- 8 673 088

## Description

L'invention concerne une installation de production de mousse contenant un additif, pour la protection contre les agressions extérieures, de manière non-permanente, d'une zone ou d'un élément sensible ou défini et/ou le marquage et/ou le camouflage d'une zone d'intérêt.

Plus précisément, l'invention concerne une installation permettant de produire une mousse, cette mousse occupe un volume défini et a une fonction de portage d'éléments ayant des propriétés particulières. Le document US 4,732,181 décrit une installation selon le préambule de la revendication 1 et un procédé selon le préambule de la revendication 15.

Dans le domaine de la sécurité, les techniques actuellement connues pour protéger un espace sensible de manière non permanente sont l'utilisation de moyens humains et/ou l'emploi de gaz irritants et/ou de lances à eau et/ou de fumigènes.

Cependant, ces techniques présentent plusieurs inconvénients.

Une protection assurée par des moyens humains pose le problème de la sécurité de ces mêmes hommes et de la disponibilité immédiate, en cas de situation de crise, d'un effectif suffisant afin d'assurer la sécurité de la zone à protéger.

La protection par l'emploi de lances à eau ne permet pas une protection continue et nécessite de très grands volumes d'eau.

Enfin l'utilisation de fumigènes, de brouillard « chargé », comme l'emploi de gaz lacrymogènes n'offre qu'une protection limitée dans le temps ainsi qu'une non-maîtrise du volume à protéger, due à la dilution rapide des gaz.

De plus les fumigènes sont inefficaces en présence de vent, en particulier à bord d'un navire.

Ainsi, il existe un besoin de développer de nouvelles techniques de protection de zones sensibles qui ne présentent pas les inconvénients ci-dessus énoncés.

Dans le domaine du marquage de zones sensibles, en particulier en cas de sauvetage, les zones sont généralement marquées au moyen de fumigènes colorés qui sont repérables par avions.

Cependant, les fumigènes utilisés se dispersent rapidement. De plus, la zone ne peut être repérée que de manière visuelle.

Dans le domaine du camouflage de zones sensibles, celles-ci sont généralement camouflées au moyen d'installations telles que des filets ou des écrans de fumées.

Cependant, ces installations présentent l'inconvénient d'être difficile à mettre en oeuvre.

Ainsi, il existe un besoin de développer de nouvelles méthodes de marquage et/ou de camouflage de zones qui n'ont pas les inconvénients ci-dessus énoncés.

En particulier, dans le domaine du camouflage, ces méthodes doivent permettre de rendre moins visibles à l'oeil et/ou aux caméras du domaine du visible et/ou des infrarouges et/ou aux radars, des individus ou des objets.

Il existe aussi un besoin de développer de nouvelles méthodes de marquage d'individu, par exemple par un marqueur biologique ou biochimique de type ADN naturel ou de synthèse. Le but de ce marquage généralement transparent, inodore et inoffensif, est de pouvoir rester ensuite sur la peau pendant une durée de six mois minimum et ne part pas au lavage.

Certaines installations consistent à projeter un brouillard de produits actifs tout autour du navire ou de l'installation.

Cependant ces installations présentent l'inconvénient de devoir être en permanence en fonctionnement pour obtenir un haut niveau de protection. En effet, le brouillard une fois projeté, se dissipe rapidement et n'est plus efficient pour assurer une protection.

Ainsi, il existe un besoin de fournir de nouvelles installations permettant d'assurer une protection et/ou un marquage et/ou un camouflage amélioré de zones sensibles et ce pour une durée importante sans avoir besoin de faire fonctionner l'installation en continu.

De plus, l'installation doit être activable rapidement.

Enfin, dans certain cas il peut être préférable que l'installation soit portable afin de pouvoir être utilisée pendant une intervention.

La demanderesse a maintenant découvert qu'une installation de production de mousse comprenant un ou plusieurs additifs particuliers permettait de répondre aux inconvénients ci-dessus énoncés.

L'invention a donc pour objet une installation de production de mousse selon la revendication 1.

L'installation selon l'invention est configurée pour produire une mousse à coefficient de foisonnement élevé, c'est-à-dire supérieur à 10, avantageusement supérieur à 20, de préférence variant de 20 à 2000, plus préférentiellement variant de 40 à 2000, encore plus préférentiellement variant de 60 à 2000, encore plus préférentiellement variant de 150 à 2000, et en particulier variant de 400 à 2000.

Cette installation permet donc de produire une mousse avec de tels coefficients de foisonnement.

Le coefficient de foisonnement peut varier en fonction de la quantité de gaz introduite dans le mélange mais peut dépendre aussi de paramètres comme le choix de l'agent tensioactif, de la concentration en cet agent, du type de buse utilisé quand des buses sont utilisées et de la maille de la grille si une grille est utilisée.

Par gaz dans la présente invention, on entend tout gaz classiquement utilisé dans les dispositifs de production de mousse.

De manière préférée dans la présente invention, le gaz est de l'air.

La mousse peut être invasive et/ou avoir une bonne tenue et/ou être très adhérente et permet l'occupation d'un vaste espace.

La mousse peut aussi bien être utilisée dans un espace fermé que dans une zone ouverte.

De plus, la mousse reste bien localisée au niveau de la zone à protéger et/ou à marquer et/ou à camoufler.

De plus, elle résiste globalement à l'action du vent.

De plus, elle délimite visuellement les zones où le ou les produits actifs principaux sont répandus, à la différence de gaz ou de brouillard chargés.

De plus, grâce à le ou les produits actifs principaux qu'elle contient, elle permet la protection et/ou le marquage et/ou le camouflage (complémentaire à la simple visualisation de la mousse) d'une zone sensible.

Avantageusement, dans le cas d'une zone à protéger, la zone peut être rendue inaccessible à des personnes non protégées au moyen d'équipements spéciaux.

Enfin, cette mousse peut être durable dans le temps et être stable de quelques minutes à quelques heures ou à plusieurs dizaines d'heures, ceci en fonction de la nature et de la teneur du ou des agents tensioactifs choisis.

L'installation selon l'invention peut aussi bien être utilisée à l'extérieur qu'à l'intérieur d'un bâtiment ainsi que sur ou dans un véhicule.

Par conséquent, l'invention a aussi pour objet un véhicule automobile, navire ou aéronef comprenant l'installation telle que décrite ci-dessus.

Enfin, l'invention a pour objet un procédé de production de mousse selon la revendication 15.

Dans la présente description, le coefficient de foisonnement est classiquement défini comme étant le rapport du volume de mousse produite sur le volume du mélange servant à former cette mousse.

L'invention comprend une installation de production de mousse comprenant :
- un premier module comprenant une sortie principale et délivrant à ladite sortie principale un mélange comprenant un liquide principal et au moins un agent tensioactif,
- un second module couplé à la sortie principale du premier module et produisant une mousse qui comprend au moins le liquide principal, le au moins un agent tensioactif et au moins un produit actif principal, à partir du mélange délivré par la sortie principale du premier module,

le mélange comprenant le au moins un produit actif principal ou
le second module comprenant en outre des moyens configurés pour que la mousse produite par le second module comprenne le au moins un produit actif principal. Ledit au moins un produit actif principal est choisi parmi les produits irritants, les produits annihilant les capacités musculaires et/ou de réflexion d'un être vivant.

L'installation produit une mousse qui présente un coefficient de foisonnement supérieur à 10, avantageusement supérieur à 20, de préférence variant de 20 à 2000, plus préférentiellement variant de 40 à 2000, encore plus préférentiellement variant de 60 à 2000, encore plus préférentiellement variant de 150 à 2000, et en particulier variant de 400 à 2000.

Le choix du coefficient de foisonnement recherché dépend du mode d'utilisation de l'installation, du lieu de génération de la mousse et de sa composition.

L'installation selon l'invention possède plusieurs modes de mise en oeuvre pour chacune des deux variantes de l'installation.

Dans une première variante de l'installation selon l'invention, le mélange délivré par la sortie principale du premier module comprend le au moins un produit actif principal.

Dans cette première variante, le premier module de l'installation selon l'invention peut comprendre différents modes de mise en oeuvre.

Dans un premier mode de mise en oeuvre de ce premier module, il comprend un réservoir unique qui comprend le liquide principal, le au moins un agent tensioactif et le au moins un produit actif principal, ce réservoir étant relié à la sortie principale du premier module.

Ce mode de mise en oeuvre « tout en un » peut notamment être utilisé dans un mode « portatif » de l'installation selon l'invention.

Dans un deuxième mode de mise en oeuvre de ce premier module, il comprend un réservoir principal qui comprend le liquide principal, ledit réservoir principal étant relié à une entrée principale d'une conduite principale, et un premier réservoir qui comprend le au moins un agent tensioactif et le au moins un produit actif principal, ledit premier réservoir étant en communication fluidique avec une première entrée secondaire de la conduite principale, ladite conduite principale étant reliée à la sortie principale du premier module.

Dans un troisième mode de mise en oeuvre de ce premier module, il comprend un réservoir principal qui comprend le liquide principal et le au moins un agent tensioactif, ledit réservoir principal étant relié à une entrée principale d'une conduite principale, et un premier réservoir qui comprend le au moins un produit actif principal, ledit premier réservoir étant en communication fluidique avec une première entrée secondaire de la conduite principale, ladite conduite principale étant reliée à la sortie principale du premier module.

Dans un quatrième mode de mise en oeuvre de ce premier module, il comprend un réservoir principal qui comprend le liquide principal et le au moins un produit actif principal, ledit réservoir principal étant relié à une entrée principale d'une conduite principale, et un premier réservoir qui comprend le au moins un agent tensioactif, ledit premier réservoir étant en communication fluidique avec une première entrée secondaire de la conduite principale, ladite conduite principale étant reliée à la sortie principale du premier module.

Dans un cinquième mode de mise en oeuvre de ce premier module, il comprend un réservoir principal qui comprend le liquide principal, ledit réservoir principal étant relié à une entrée principale d'une conduite principale, un premier réservoir qui comprend le au moins un agent tensioactif, ledit premier réservoir étant en communication fluidique avec une première entrée secondaire de la conduite principale, et un deuxième réservoir qui comprend le au moins un produit actif principal, ledit deuxième réservoir étant en communication fluidique avec une deuxième entrée secondaire de la conduite principale, ladite conduite principale étant reliée à la sortie principale du premier module.

Dans un sixième mode de mise en oeuvre de ce premier module, il comprend une entrée principale d'une conduite principale reliée à une arrivée de liquide principal, un premier réservoir qui comprend le au moins un agent tensioactif et le au moins un produit actif principal, ledit premier réservoir étant en communication fluidique avec une première entrée secondaire de la conduite principale, ladite conduite principale étant reliée à la sortie principale du premier module.

Dans un septième mode de mise en oeuvre de ce premier module, il comprend une entrée principale d'une conduite principale reliée à une arrivée de liquide principal, un premier réservoir qui comprend le au moins un agent tensioactif, ledit premier réservoir étant en communication fluidique avec une première entrée secondaire de la conduite principale, et un deuxième réservoir qui comprend le au moins un produit actif principal, ledit deuxième réservoir étant en communication fluidique avec une deuxième entrée secondaire de la conduite principale, ladite conduite principale étant reliée à la sortie principale du premier module.

Dans une seconde variante de l'installation selon l'invention, le second module comprend en outre des moyens configurés pour que la mousse produite par le second module comprenne le au moins un produit actif principal.

Dans cette seconde variante, le premier module de l'installation selon l'invention peut comprendre différents modes de mise en oeuvre.

Dans un premier mode de mise en oeuvre de ce premier module, il comprend un réservoir unique qui comprend le liquide principal, le au moins un agent tensioactif et optionnellement au moins un produit actif secondaire, ce réservoir étant relié à la sortie principale du premier module.

Ce mode de mise en oeuvre « tout en un » peut notamment être utilisé dans un mode « portatif » de l'installation selon l'invention.

Dans un deuxième mode de mise en oeuvre de ce premier module, il comprend un réservoir principal qui comprend le liquide principal, et optionnellement au moins un produit actif secondaire, ledit réservoir principal étant relié à un entrée principale d'une conduite principale, et un premier réservoir qui comprend le au moins un agent tensioactif, ledit premier réservoir étant en communication fluidique avec une première entrée secondaire de la conduite principale, ladite conduite principale étant reliée à la sortie principale du premier module.

Dans un troisième mode de mise en oeuvre de ce premier module, il comprend un réservoir principal qui comprend le liquide principal, ledit réservoir principal étant relié à un entrée principale d'une conduite principale, et un premier réservoir qui comprend le au moins un agent tensioactif et optionnellement au moins un produit actif secondaire, ledit premier réservoir étant en communication fluidique avec une première entrée secondaire de la conduite principale, ladite conduite principale étant reliée à la sortie principale du premier module.

Dans un quatrième mode de mise en oeuvre de ce premier module, il comprend un réservoir principal qui comprend le liquide principal et le au moins un agent tensioactif, ledit réservoir principal étant relié à un entrée principale d'une conduite principale, et un premier réservoir qui comprend au moins un produit actif secondaire, ledit premier réservoir étant en communication fluidique avec une première entrée secondaire de la conduite principale, ladite conduite principale étant reliée à la sortie principale du premier module.

Dans un cinquième mode de mise en oeuvre de ce premier module, il comprend un réservoir principal qui comprend le liquide principal, ledit réservoir principal étant relié à une entrée principale d'une conduite principale, un premier réservoir qui comprend le au moins un agent tensioactif, ledit premier réservoir étant en communication fluidique avec une première entrée secondaire de la conduite principale, et un deuxième réservoir qui comprend au moins un produit actif secondaire, ledit deuxième réservoir étant en communication fluidique avec une deuxième entrée secondaire de la conduite principale, ladite conduite principale étant reliée à la sortie principale du premier module.

Dans un sixième mode de mise en oeuvre de ce premier module, il comprend une entrée principale d'une conduite principale reliée à une arrivée de liquide principal, un premier réservoir qui comprend le au moins un agent tensioactif et optionnellement au moins un produit actif secondaire, ledit premier réservoir étant en communication fluidique avec une première entrée secondaire de la conduite principale, ladite conduite principale étant reliée à la sortie principale du premier module.

Dans un septième mode de mise en oeuvre de ce premier module, il comprend une entrée principale d'une conduite principale reliée à une arrivée de liquide principal, un premier réservoir qui comprend le au moins un agent tensioactif, ledit premier réservoir étant en communication fluidique avec une première entrée secondaire de la conduite principale, et un deuxième réservoir qui comprend au moins un produit actif secondaire, ledit deuxième réservoir étant en communication fluidique avec une deuxième entrée secondaire de la conduite principale, ladite conduite principale étant reliée à la sortie principale du premier module.

Aussi bien dans la première que dans la seconde variante de cette installation, le réservoir unique du premier module ou le réservoir principal du premier module peuvent avoir de préférence une entrée reliée à un système pyrotechnique, ou un système de stockage et/ou de production de gaz comprimé, afin de mettre sous pression l'installation.

Enfin, que ce soit dans la première ou dans la seconde variante de cette installation, le réservoir unique du premier module ou le réservoir principal du premier module peuvent être sous une pression supérieure à la pression atmosphérique.

De préférence, le système de stockage et/ou de production de gaz comprimé comprend un compresseur et/ou une bouteille de gaz sous pression.

Le second module de l'installation selon l'invention est configuré pour produire une mousse à coefficient de foisonnement supérieur à 10, avantageusement supérieur à 20, de préférence variant de 20 à 2000, plus préférentiellement variant de 40 à 2000, encore plus préférentiellement variant de 60 à 2000, encore plus préférentiellement variant de 150 à 2000, et en particulier variant de 400 à 2000.

Le second module de l'installation selon l'invention peut avoir différents modes de mise en oeuvre.

Dans un premier mode de mise en oeuvre de ce second module, il comprend une entrée principale reliée à la sortie principale du premier module, ladite entrée principale étant reliée à au moins une buse principale, une grille étant disposée en aval de ladite au moins une buse principale, et, en amont de ladite au moins une buse principale, des moyens configurés pour produire un souffle dans le sens de l'écoulement du mélange présent à l'entrée principale du second module.

Par souffle au sens de la présente invention, on entend un courant de gaz.

Dans un deuxième mode de mise en oeuvre de ce second module, il comprend une entrée principale reliée à la sortie principale du premier module, ladite entrée principale étant reliée à au moins une buse principale, une section d'admission de gaz en forme de carter étant disposée en aval de ladite au moins une buse principale, l'axe longitudinal de cette section d'admission étant parallèle ou identique à l'axe de projection de ladite au moins une buse principale, et en aval de cette section d'admission, une grille disposée sur la sortie de la section d'admission.

De manière préférée, le carter peut être annulaire.

Dans ce mode de réalisation, la grille peut être de forme tronconique, sous la forme d'une surface sphérique, sous la forme d'une surface elliptique, de forme plane ou de toute forme intermédiaire.

Dans un troisième mode de mise en oeuvre de ce second module, il comprend une entrée principale reliée à la sortie principale du premier module, ladite entrée principale étant reliée à un réservoir final, ledit réservoir final comprenant un système de barbotage.

De préférence, le système de barbotage dans le deuxième module de l'installation selon l'invention est réparti de manière uniforme sur toute la surface du fond du réservoir final.

Avantageusement, une grille peut être située au dessus du réservoir final ou a la sortie d'un conduit canalisant la mousse.

Le système de barbotage peut avantageusement comprendre une entrée, ladite entrée étant reliée à un système pyrotechnique, ou à un système de stockage et/ou de production de gaz comprimé tel que défini précédemment.

Le système de barbotage peut aussi être avantageusement relié à un réservoir sous une pression supérieure à la pression atmosphérique.

Dans le premier et le deuxième mode de mise en oeuvre de ce second module, le maillage de la grille n'est pas limitatif dans l'installation selon l'invention.

Le choix de la dimension de la maille de la grille peut dépendre notamment de la taille des bulles recherchée, de la vitesse du flux de gaz qui va constituer les bulles de mousse, de la nature de l'agent tensioactif et de la viscosité du mélange principal.

Cependant, de manière générale, le maillage sera de préférence compris entre 1 et 4 mm de diamètre. Il peut aussi être réalisé avec un nid d'abeille.

Dans le premier et le deuxième mode de mise en oeuvre de ce second module, les moyens configurés pour produire un souffle sont configurés pour produire une mousse de coefficient de foisonnement supérieur à 10, avantageusement supérieur à 20, de préférence variant de 20 à 2000, plus préférentiellement variant de 40 à 2000, encore plus préférentiellement variant de 60 à 2000, encore plus préférentiellement variant de 150 à 2000, et en particulier variant de 400 à 2000.

Dans le premier et le deuxième mode de mise en oeuvre de ce second module, les moyens configurés pour produire un souffle comprennent généralement un ventilateur ou un système pyrotechnique.

Dans l'installation selon l'invention, le système pyrotechnique peut être tout dispositif classiquement utilisé pour le déclenchement rapide de souffle.

Dans l'installation selon l'invention, le système pyrotechnique comprend généralement une composition explosive.

De manière avantageuse, la composition explosive peut être toute composition explosive connue de l'homme du métier.

De manière avantageuse, la conduite principale du premier module comprend une vanne située en aval de l'entrée principale du premier module ou du réservoir principal du premier module ou du réservoir unique du premier module et en amont, si elles sont présentes, du ou des entrées secondaires.

Cette ou ces vannes permettent de contrôler l'alimentation en fluide de l'installation.

Dans un mode de réalisation particulier de l'installation selon l'invention, la mousse produite par le second module comprend en outre au moins un additif.

Cet additif peut être en particulier un agent stabilisant permettant de conférer une stabilité élevée dans le temps à la mousse.

Cet additif peut être présent, par exemple dans le réservoir unique, le réservoir principal, le premier réservoir et/ou le second réservoir, s'ils sont présents dans l'installation selon l'invention.

De manière avantageuse dans ce mode de réalisation particulier de l'installation, le premier module peut comprendre au moins un troisième réservoir qui comprend le au moins un additif, ledit troisième réservoir étant en communication fluidique avec une troisième entrée secondaire de la conduite principale du premier module.

Le au moins un additif est une gomme arabique.

Le au moins un additif peut permettre notamment de stabiliser la mousse et/ou le au moins un produit actif principal et éventuellement le au moins un produit actif secondaire.

De manière avantageuse, le mélange délivré en sortie principale du premier module a une teneur en additif, s'il est présent, variant de 0,0001 à 10 % en poids, de préférence de 0,001 à 1 % en poids, par rapport au poids total du mélange délivré en sortie principale du premier module.

L'emplacement des entrées secondaires sur la conduite principale n'a pas d'importance dans l'installation telle que revendiquée.

Par exemple, la deuxième entrée secondaire peut être aussi bien en amont qu'en aval de la première entrée secondaire.

De la même manière, la troisième entrée secondaire peut très bien se situer en amont ou en aval des première et deuxième entrées secondaires ou se situer entre ces deux entrées secondaires.

De manière avantageuse, les communications fluidiques des réservoirs avec les entrées secondaires s'effectuent au moyen de mélangeurs.

Ces mélangeurs permettent d'obtenir un liquide homogène, dans le cas où le mélange est totalement liquide, ou une distribution homogène du gaz et/ou des particules solides dans le liquide dans le cas où le mélange est hétérogène.

De manière avantageuse, l'installation peut comprendre un système de pilotage commandant le fonctionnement, lorsqu'ils sont présents, de la vanne et/ou des mélangeurs et/ou des moyens configurés pour produire un souffle.

Le système de pilotage peut être manuel, semi-automatique, ou automatique.

De manière préférée, lorsque le système de pilotage est manuel, il comprend un ou plusieurs boutons poussoirs.

De manière préférée, lorsque le système de pilotage est semi-automatique, il comprend un ou plusieurs automates industriels programmables, une ou plusieurs consoles de commandes et un ou plusieurs contacteurs permettant l'activation de la production de la mousse.

De manière préférée, le système de pilotage peut être automatiquement piloté par un dispositif sensible, un système de vidéo surveillance, une cellule de détection infrarouge ou une caméra infrarouge par exemple.

De manière avantageuse, les moyens configurés pour que la mousse produite par le second module comprenne le au moins un produit actif principal, peuvent comprendre un dispositif d'addition qui comprend un réservoir supplémentaire qui comprend le au moins un produit actif principal, le réservoir supplémentaire étant relié à au moins un moyen apte à ajouter le au moins un produit actif principal au mélange présent à l'entrée principale du second module.

Dans les cas particuliers où l'entrée principale du second module est reliée à au moins une buse principale, le moyen apte à ajouter le au moins un produit actif principal au mélange présent à l'entrée principale du second module peut comprendre au moins une buse supplémentaire, l'écoulement du au moins un produit actif principal étant dans le même sens que l'écoulement du mélange présent à l'entrée principale du second module.

Ce dispositif d'addition permet avantageusement d'ajouter aux bulles de mousse en formation un gaz et/ou un liquide en suspension tel qu'un brouillard, et/ou un solide, tel qu'une fumée, emprisonné à l'intérieur des bulles.

De manière avantageuse, l'installation selon l'invention peut comprendre en outre un ou plusieurs réservoirs additionnels configurés pour recevoir chacun un ou plusieurs additifs additionnels, le ou lesdits réservoirs additionnels étant chacun en communication fluidique avec une entrée secondaire additionnelle de la conduite principale.

Le ou les entrées secondaires additionnelles peuvent être placées à n'importe quel endroit de la conduite principale, par exemple, en amont ou en aval des entrées secondaires.

L'installation selon l'invention permet entre autres, de protéger des bâtiments terrestres sensibles tels que des postes de gestion de crise, des noeuds logistiques, des centres de traitement de données, des prisons, des centres de communications, tous types de points névralgiques et d'intérêts vitaux au niveau régional et/ou national, et/ou isolés (éoliennes, phares, avions, bijouteries, banques, établissements gouvernementaux,...).

L'installation peut aussi protéger et être sur des éléments mobiles tels que des navires de toutes tailles mais aussi des édifices flottants ou fixes tels que des plateformes, des barges, des hydroliennes, des barrages, des véhicules de transports de matériels/denrées/personnels, des véhicules VIP, des convois, des véhicules de transferts de fond de valeurs, de transfèrement judiciaire, des véhicules sur roues, chenilles ou rails.

L'installation peut également être destinée à être portée par un être humain.

L'installation peut également être destinée à camoufler un objet (par exemple un véhicule) ou un être humain.

De manière avantageuse, le liquide principal de l'installation selon l'invention est choisi parmi une eau douce, une eau de mer, une huile, un alcool et/ou un mélange de ces liquides.

De manière avantageuse, l'arrivée de liquide principal peut comprendre un circuit d'eau urbain, une pompe prélevant directement l'eau depuis une source naturelle, tel que de l'eau de mer par exemple.

Généralement, l'arrivée de liquide principal est reliée à un réseau sous pression supérieure à la pression atmosphérique, ce qui permet le déplacement du liquide principal dans l'installation.

Le réservoir unique et le réservoir principal de l'installation selon l'invention peuvent avantageusement être sous une pression supérieure à la pression atmosphérique.

Cette pression peut notamment être obtenue au moyen d'un système pyrotechnique, ou au moyen d'un système de stockage et/ou de production de gaz comprimé tel que défini précédemment.

De manière avantageuse, le au moins un agent tensioactif présent dans l'installation selon l'invention peut être choisi parmi ceux à base protéiniques obtenus par hydrolyse (décomposition) de protéines animales ou végétales ou ceux à base synthétiques issus de l'industrie chimique ou encore toute molécule capable de générer une mousse.

On choisira de préférence le au moins un agent tensioactif parmi les tensioactifs ioniques et les tensioactifs non-ioniques, et de préférence parmi les tensioactifs ioniques qui possèdent une chaîne hydrophobe linéaire comme le dodécyl-sulfate, avec ajout de l'ordre de 1 % de co-tensioactifs non-ioniques comme les monoéthanolamides de chaîne linéaire en C₁₂ à C₁₄, ou les isopropanol amides, ou encore les diéthanolamides.

Des mélanges de différents types de tensioactif (anionique, cationique, amphotère) sont possibles suivant les caractéristiques de la mousse désirée (foisonnement, décantation).

Le choix du tensioactif peut être effectué en fonction de l'application recherchée pour la mousse produite.

De manière avantageuse, la teneur en agent tensioactif dans le mélange délivré en sortie principale du premier module varie de 1 à 10 % en poids, et de préférence de 2 à 7 % en poids, par rapport au poids total du mélange présent en sortie principale de la conduite principale.

Selon un premier mode préféré, on entend par produit actif un composé qui altère les capacités physiques d'un être vivant, en particulier d'un être humain.

De manière avantageuse dans ce premier mode préféré, le au moins un produit actif secondaire, s'il est présent, peut être choisi parmi les produits irritants et les produits annihilant les capacités musculaires et/ou de réflexion d'un être vivant.

Selon un deuxième mode préféré, on entend par produit actif secondaire un composé qui confère un marquage à l'élément avec lequel il est en contact.

De manière avantageuse dans ce deuxième mode préféré, le au moins un produit actif secondaire, s'il est présent, peut être choisi parmi les colorants, les produits fluorescents et les marqueurs chimiques et/ou biologiques.

Selon un troisième mode préféré, on entend par produit actif secondaire un composé qui confère un camouflage à une zone particulière.

De manière avantageuse dans ce troisième mode préféré, le au moins un produit actif secondaire, s'il est présent, peut être choisi parmi les colorants.

Selon un quatrième mode préféré, on entend par produit actif secondaire un composé choisi parmi les gaz et les réflecteurs de radar.

Dans l'installation de production de mousse selon l'invention, le au moins produit actif principal est choisi parmi les produits irritants, les produits annihilant les capacités musculaires et/ou de réflexion d'un être vivant.

A titre de produit irritant ou annihilant les capacités musculaires et/ou de réflexion d'un être vivant, on pourra notamment utiliser l'oléorésine de capsicum (OC) ou un de ses dérivés, le chlorobenzylidène malononitrile (CS), la chloroacétophénone (CN), le cyanure de bromobenzyle (CA), la dibenzo(b,f,)-1,4-oxazépine (CR), le nonivamide (PAVA), la capsaïcine, la dihydrocapsaïcine, la nordihydrocapsaïcine, l'homodihydrocapsaïcine et l'homocapsaïcine.

Selon l'invention, le ou les produits irritants et le ou les produits annihilant les capacités musculaires et/ou de réflexion d'un être vivant sont sous forme encapsulés dans de la gomme arabique, et en particulier l'oléorésine de capsicum ou un de ses dérivés et le nonivamide.

A titre de colorant, on pourra notamment utiliser ceux présentant une forte solubilité dans le liquide principal, de préférence l'eau et une stabilité chimique importante.

On pourra notamment privilégier le bleu de méthylène, l'Éosine Y (rouge), le Radamine WT (rouge), la Falavine FF de Lissamine (jaune) et les colorants alimentaires comme par exemple le bleu patenté V[E131], l'azorubine[E122] (rouge) et le jaune de quinoléïne[E104] (jaune).

A titre de produit fluorescent, on pourra notamment utiliser le sulfure de zinc, la wurtzite, de formule de base ZnS associé à un métal lourd, le scheelite (CaWO₄), un autre sulfure alcalin ou alcalinoterreux comme CaS par exemple.

A titre de marqueur chimique et/ou biologique, on pourra notamment utiliser de l'ADN de synthèse composé d'un enchaînement des quatre acides nucléiques qui le composent : adénine, thymine, guanine et cytosine.

A titre de réflecteur radar, on pourra notamment utiliser des composés visibles par des radars tels que de fines gouttes d'huile contenant de la poudre métallique très fine.

De manière préférée, le au moins un produit actif principal et/ou le au moins un produit actif secondaire éventuel sont choisis parmi les produits irritants et les produits annihilant les capacités musculaires et/ou de réflexion d'un être vivant, et de préférence, le au moins un produit actif principal et/ou le au moins un produit actif secondaire éventuel sont la capsaïcine, l'oléorésine de capsicum ou un de ses dérivés et le nonivamide.

De manière avantageuse, le au moins un produit actif principal et le au moins un produit actif secondaire éventuel ont chacun une teneur variant de 0,1 à 10 % en poids, de préférence de 0,5 à 3 % en poids, par rapport au poids total du mélange délivré en sortie principale du premier module.

De manière avantageuse, le ou les produits actifs principaux peuvent avoir une durée d'efficacité plus courte, ou aussi longue que la durée d'existence de la mousse.

Le ou les réservoirs additionnels de l'installation selon l'invention, s'ils y sont présents, comprennent chacun un ou plusieurs additifs additionnels.

Le ou lesdits additifs additionnels peuvent être choisis parmi ceux cités précédemment à titre d'agent tensioactif, de produit actif principal et d'additif.

De manière avantageuse, le réservoir supplémentaire de l'installation selon l'invention, s'il est présent, comprend un fluide supplémentaire choisi parmi un gaz, un liquide, avec optionnellement de fines particules de produit actif.

L'invention a également pour objet une mousse susceptible d'être produite par l'installation telle que définie ci-dessus, présentant avantageusement un coefficient de foisonnement supérieur à 10, très avantageusement supérieur à 20, de préférence variant de 20 à 2000, plus préférentiellement variant de 40 à 2000, encore plus préférentiellement variant de 60 à 2000, encore plus préférentiellement variant de 150 à 2000, et en particulier variant de 400 à 2000.

De manière avantageuse, le au moins un produit actif principal se situe dans la paroi des bulles de la mousse selon l'invention et/ou sous forme de gaz à l'intérieur des bulles de la mousse et/ou sous forme de fines particules de liquide ou de solide en suspension à l'intérieur des bulles de la mousse.

Lorsque l'additif principal est sous forme liquide, il se situe dans la paroi des bulles de la mousse.

Lorsque l'additif principal est sous forme de gaz, de fines particules de liquide ou de solide en suspension il se situe à l'intérieur des bulles de la mousse.

L'invention a également pour objet l'utilisation de l'installation décrite précédemment pour protéger des bâtiments terrestres sensibles tels que des postes de gestion de crise, des noeuds logistiques, des centres de traitement de données, des prisons, des centres de communications, tous types de points névralgiques et d'intérêt vitaux au niveau régional et/ou national, et/ou isolés (éoliennes, phares, avions, bijouteries, banques, établissements gouvernementaux,...).

L'installation peut aussi être utilisée pour protéger et être sur des éléments mobiles tels que des navires de toutes tailles mais aussi des édifices flottants ou fixes tels que des plateformes, des barges, des hydroliennes, des barrages, des véhicules de transports de matériels/denrées/personnels, des véhicules VIP, des convois, des véhicules de transferts de fond de valeurs, de transfèrement judiciaire, des véhicules sur roues, chenilles ou rails.

L'installation peut également être destinée à être portée par un être humain.

L'installation peut également être destinée à camoufler un objet (par exemple un véhicule) ou un être humain.

L'invention a également pour objet un véhicule automobile, navire ou aéronef comprenant l'installation telle que définie précédemment.

L'invention a enfin pour objet un procédé de production d'une mousse selon la revendication 15.

D'autres avantages et caractéristiques de l'invention apparaitront plus clairement à l'examen de la description détaillée des différents modes de mise en oeuvre de l'invention, nullement limitatifs, et des dessins annexés, sur lesquels :
- les figures 1a à 1e présentent de manière schématique différents modes de réalisation du premier module ;
- les figures 2a à 2e présentent de manière schématique différents modes de réalisation du premier module ;
- les figures 3a à 3c présentent de manière schématique différents modes de réalisation du second module ;
- la figure 4 présente de manière schématique une installation de production de mousse selon un premier mode de réalisation ;
- la figure 5 présente de manière schématique une installation de production de mousse selon un deuxième mode de réalisation ;
- la figure 6 présente de manière schématique une installation de production de mousse selon un troisième mode de réalisation ;
- la figure 7 présente de manière schématique, une installation de production de mousse selon un quatrième mode de réalisation ;
- la figure 8 présente de manière schématique, une installation de production de mousse selon un cinquième mode de réalisation ;
- la figure 9 présente de manière schématique, une installation de production de mousse selon un cinquième mode de réalisation.

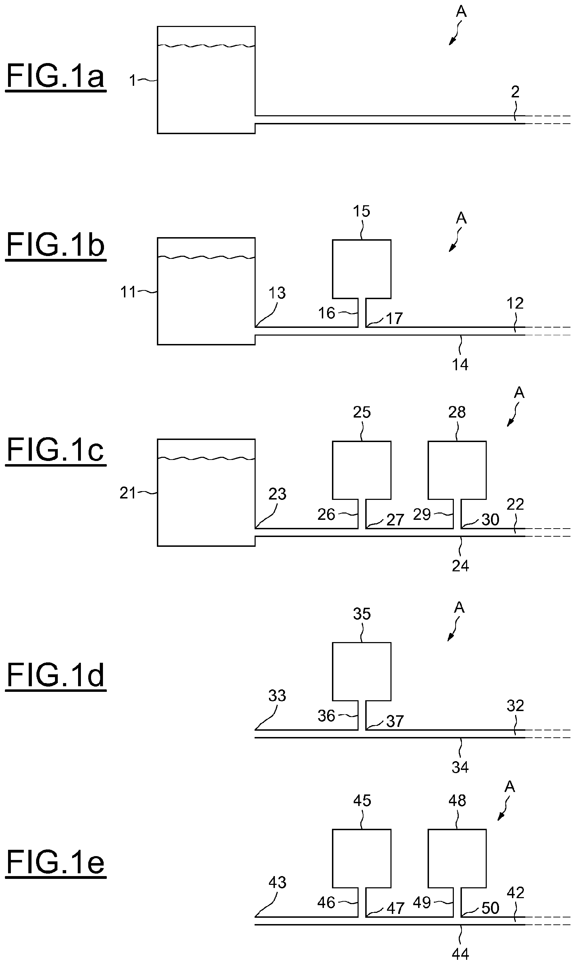

Les figures 1a à 1e décrivent les différents modes de mise en oeuvre d'un premier module A dans la première variante de l'installation selon l'invention.

Dans la figure 1a, le premier module comprend un réservoir unique 1 qui comprend le liquide principal, le au moins un agent tensioactif et le au moins un produit actif principal, ce réservoir 1 étant relié à la sortie principale 2 du premier module.

Dans la figure 1b, le premier module comprend un réservoir principal 11 qui comprend le liquide principal, et optionnellement le au moins un agent tensioactif ou le au moins un produit actif principal, ledit réservoir principal 11 étant relié à un entrée principale 13 d'une conduite principale 14, et un premier réservoir 15 qui comprend le au moins un agent tensioactif et/ou le au moins un produit actif principal, ledit premier réservoir 15 étant en communication fluidique 16 avec une première entrée secondaire 17 de la conduite principale 14, ladite conduite principale étant reliée à la sortie principale 12 du premier module.

Dans la figure 1c, le premier module comprend un réservoir principal 21 qui comprend le liquide principal, ledit réservoir principal 21 étant relié à une entrée principale 23 d'une conduite principale 24, un premier réservoir 25 qui comprend le au moins un agent tensioactif, ledit premier réservoir 25 étant en communication fluidique 26 avec une première entrée secondaire 27 de la conduite principale 24, et un deuxième réservoir 28 qui comprend le au moins un produit actif principal, ledit deuxième réservoir étant en communication fluidique 29 avec une deuxième entrée secondaire 30 de la conduite principale 24, ladite conduite principale 24 étant reliée à la sortie principale 22 du premier module.

Dans la figure 1d, le premier module comprend une entrée principale 33 d'une conduite principale 34 reliée à une arrivée de liquide principal, un premier réservoir 35 qui comprend le au moins un agent tensioactif et le au moins un produit actif principal, ledit premier réservoir 35 étant en communication fluidique 36 avec une première entrée secondaire 37 de la conduite principale 34, ladite conduite principale 34 étant reliée à la sortie principale 32 du premier module.

Dans la figure 1e, le premier module comprend une entrée principale 43 d'une conduite principale 44 reliée à une arrivée de liquide principal, un premier réservoir 45 qui comprend le au moins un agent tensioactif, ledit premier réservoir 45 étant en communication fluidique 46 avec une première entrée secondaire 47 de la conduite principale 44, et un deuxième réservoir 48 qui comprend le au moins un produit actif principal, ledit deuxième réservoir 48 étant en communication fluidique 49 avec une deuxième entrée secondaire 50 de la conduite principale 44, ladite conduite principale 44 étant reliée à la sortie principale 42 du premier module.

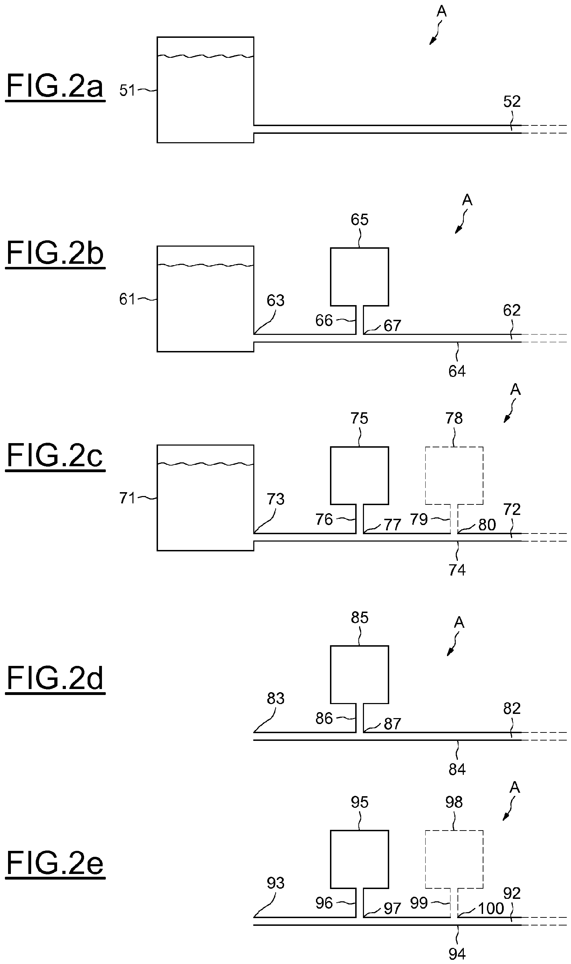

Les figures 2a à 2e décrivent les différents modes de mise en oeuvre d'un premier module A dans la seconde variante de l'installation selon l'invention.

Dans la figure 2a, le premier module comprend un réservoir unique 51 qui comprend le liquide principal, le au moins un agent tensioactif et optionnellement au moins un produit actif secondaire, ce réservoir 51 étant relié à la sortie principale 52 du premier module.

Dans la figure 2b, le premier module comprend un réservoir principal 61 qui comprend le liquide principal, et optionnellement le au moins un agent tensioactif ou au moins un produit actif secondaire, ledit réservoir principal 61 étant relié à un entrée principale 63 d'une conduite principale 64, et un premier réservoir 65 qui comprend le au moins un agent tensioactif et/ou optionnellement le au moins un produit actif secondaire, ledit premier réservoir 65 étant en communication fluidique 66 avec une première entrée secondaire 67 de la conduite principale 64, ladite conduite principale 64 étant reliée à la sortie principale 62 du premier module.

Dans la figure 2c, le premier module comprend un réservoir principal 71 qui comprend le liquide principal, ledit réservoir principal étant relié à une entrée principale 73 d'une conduite principale 74, un premier réservoir 75 qui comprend le au moins un agent tensioactif, ledit premier réservoir 75 étant en communication fluidique 76 avec une première entrée secondaire 77 de la conduite principale 74, et optionnellement un deuxième réservoir 78 qui comprend au moins un produit actif secondaire, ledit deuxième réservoir 78 étant en communication fluidique 79 avec une deuxième entrée secondaire 80 de la conduite principale 74, ladite conduite principale 74 étant reliée à la sortie principale 72 du premier module.

Dans la figure 2d, le premier module comprend une entrée principale 83 d'une conduite principale 84 reliée à une arrivée de liquide principal, un premier réservoir 85 qui comprend le au moins un agent tensioactif et optionnellement au moins un produit actif secondaire, ledit premier réservoir 85 étant en communication fluidique 86 avec une première entrée secondaire 87 de la conduite principale 84, ladite conduite principale 84 étant reliée à la sortie principale 82 du premier module,

Dans la figure 2e, le premier module comprend une entrée principale 93 d'une conduite principale 94 reliée à une arrivée de liquide principal, un premier réservoir 95 qui comprend le au moins un agent tensioactif, ledit premier réservoir 95 étant en communication fluidique 96 avec une première entrée secondaire 97 de la conduite principale, et optionnellement un deuxième réservoir 98 qui comprend au moins un produit actif secondaire, ledit deuxième réservoir 98 étant en communication fluidique 99 avec une deuxième entrée secondaire 100 de la conduite principale 94, ladite conduite principale 94 étant reliée à la sortie principale 92 du premier module.

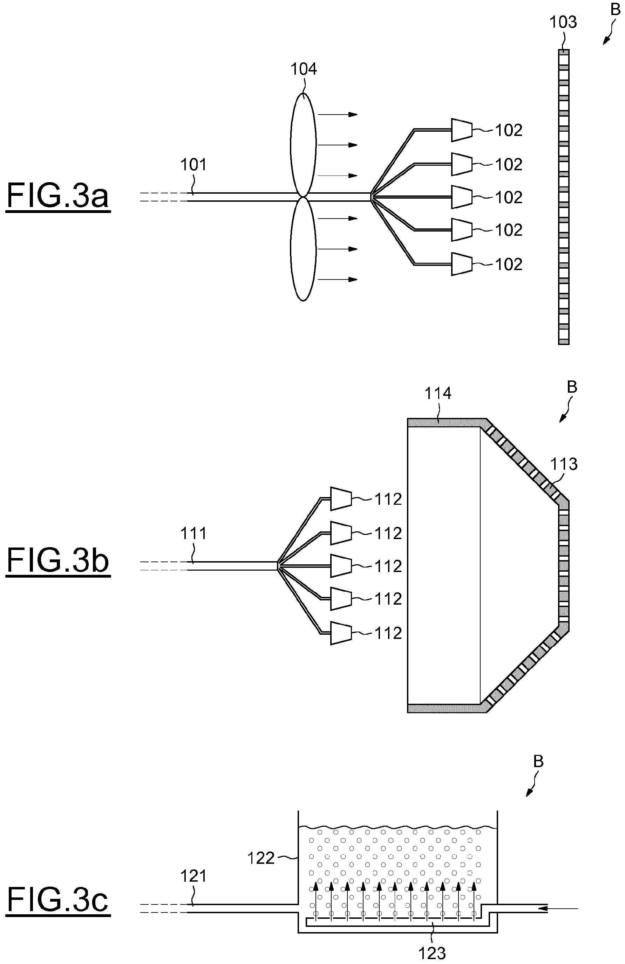

Les figures 3a à 3c décrivent différents modes de mise en oeuvre d'un second module B.

Dans la figure 3a, le second module comprend une entrée principale 101 reliée à la sortie principale du premier module, ladite entrée principale 101 étant reliée à au moins une buse principale 102, une grille 103 étant disposée en aval de ladite au moins une buse principale 102, et, en amont de ladite au moins une buse principale 102, des moyens configurés pour produire un souffle 104 dans le sens de l'écoulement du mélange présent à l'entrée principale 101 du second module.

Dans la figure 3b, le second module comprend une entrée principale 111 reliée à la sortie principale du premier module, ladite entrée principale 111 étant reliée à au moins une buse principale 112, une section d'admission 114 de gaz en forme de carter étant disposée en aval de ladite au moins une buse principale 112, l'axe longitudinal de cette section d'admission 114 étant parallèle ou identique à l'axe de projection de ladite au moins une buse principale 112, et en aval de cette section d'admission, une grille 113 disposée sur la sortie de la section d'admission 114.

Dans le cas de la figure 3b, ce mode de réalisation du second module fonctionne comme suit : le mélange en sortie principale du premier module arrive à l'entrée principale du second module 111 et est projeté au moyen d'au moins une buse principale 112 à travers la section d'admission 114. Ceci produit un effet Venturi qui apporte un gaz au mélange. L'ensemble est ensuite projeté sur une grille 113 qui permet de produire les bulles de mousse.

Dans la figure 3c, le second module comprend une entrée principale 121 reliée à la sortie principale du premier module, ladite entrée principale 121 étant reliée à un réservoir final 122, ledit réservoir final comprenant un système de barbotage 123.

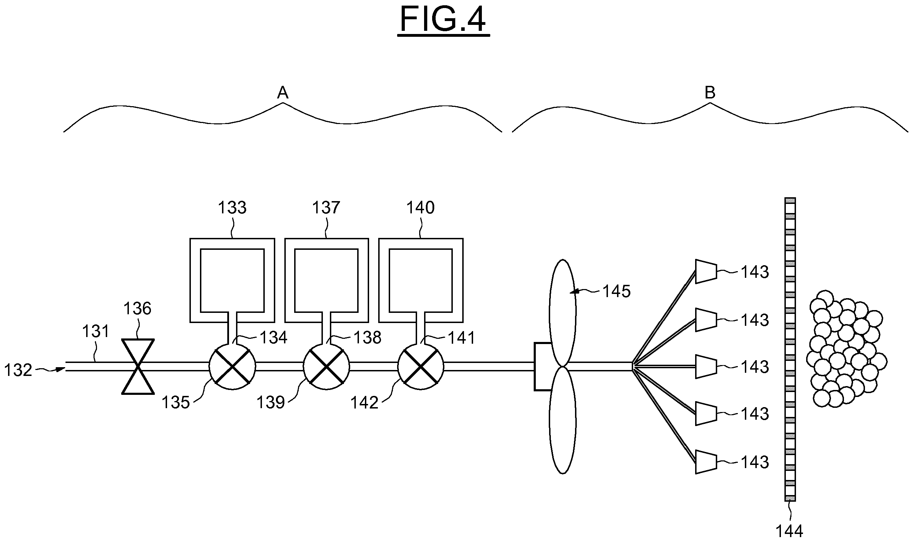

La figure 4 présente un premier mode de mise en oeuvre de l'installation de production de mousse selon l'invention.

Cette installation comprend une conduite principale 131 ayant une entrée principale 132. Cette entrée principale est reliée à une arrivée de liquide principal. L'arrivée de liquide principal pouvant être tel que définie précédemment.

L'installation comprend un premier réservoir 133 qui comprend le au moins un agent tensioactif tel que défini précédemment.

Le premier réservoir 133 est en communication fluidique avec une première entrée secondaire 134 de la conduite principale 131.

La communication fluidique s'effectue au moyen d'un mélangeur 135.

De manière préférée, le mélangeur 135 fonctionne avec un système de succion par effet Venturi ou par une pompe d'injection.

En aval de ce premier mélangeur 135, une vanne 136 est présente sur la conduite principale 131.

Cette installation comprend un deuxième réservoir 137 qui comprend le au moins un produit actif principal tel que défini précédemment.

Le deuxième réservoir 137 est en communication fluidique avec une deuxième entrée secondaire 138 de la conduite principale 131. Cette deuxième entrée secondaire 138 se situe en aval de la première entrée secondaire 134.

La communication fluidique s'effectue au moyen d'un mélangeur 139.

De manière préférée, le mélangeur 139 fonctionne avec un système de succion par effet Venturi ou par une pompe d'injection.

Cette installation selon la figure 4 comprend en outre un troisième réservoir 140 qui comprend le au moins un additif tel que défini précédemment.

Le troisième réservoir 140 étant en communication fluidique avec une troisième entrée secondaire 141 de la conduite principale 131. Cette troisième entrée secondaire 141 se situe en aval de la deuxième entrée secondaire 138.

La communication fluidique s'effectue au moyen d'un mélangeur 142.

De manière préférée, le mélangeur 142 fonctionne avec un système de succion par effet Venturi ou par une pompe d'injection.

La conduite principale 141 est reliée à au moins une buse principale 143. Le nombre de buses principales n'est pas limitatif dans l'installation selon l'invention. Cependant, ce nombre est de préférence compris entre 1 et 12.

Une grille 144 se situe en aval de ladite au moins une buse principale 143.

Les moyens configurés pour produire un souffle 145 se situent en amont de ladite au moins une buse principale 143 afin de produire un souffle dans le sens de l'écoulement du mélange présent à l'entrée principale du second module. Dans cette figure 4, les moyens pour produire un souffle comprennent un ventilateur.

Pour faire fonctionner l'installation selon la figure 4, l'entrée principale 132 est reliée à une arrivée de liquide principal. Ce liquide principal passe ensuite à travers la vanne 136 pour atteindre le premier mélangeur 135. Le liquide principal est ainsi mélangé à le au moins un agent tensioactif présent dans le premier réservoir 133 et relié à la conduite principale 131 au moyen de la première entrée secondaire 134.

Le mélange ainsi obtenu arrive dans le deuxième mélangeur 139. Le mélange est ainsi brassé avec le au moins un produit actif principal présent dans le deuxième réservoir 137 et relié à la conduite principale 131 au moyen de la deuxième entrée secondaire 138.

Le mélange ainsi obtenu arrive dans le troisième mélangeur 142. Le mélange est brassé avec le au moins un additif présent dans le troisième réservoir 140 et relié à la conduite principale 131 au moyen de la troisième entrée secondaire 141.

Le mélange ainsi obtenu est projeté au moyen d'au moins une buse principale 143 sur une grille 144. Des moyens configurés pour produire un souffle 145 produisent les bulles de la mousse en projetant du gaz dans le mélange présent sur la grille 144.

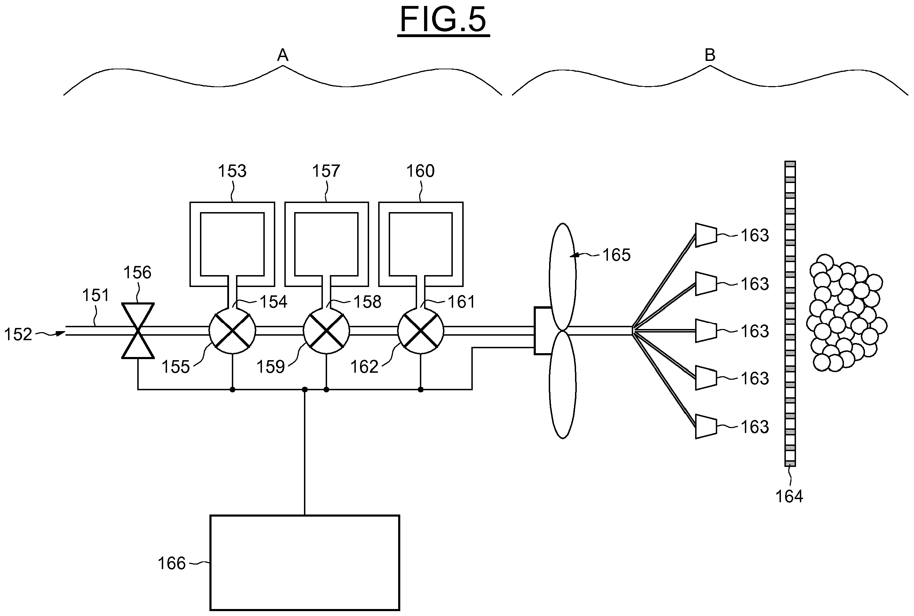

La figure 5 présente un deuxième mode de mise en oeuvre de l'installation de production de mousse selon l'invention.

Cette installation reprend les éléments de l'installation selon la figure 4. Par conséquent, les références des éléments communs dans la figure 5 sont les mêmes que pour la figure 4 incrémentés de 20.

Cette installation comprend les éléments 151 à 165 qui sont agencés et qui fonctionnent de la même manière que précédemment pour les éléments correspondants dans la figure 4.

Cette installation selon la figure 5 comprend en outre un système de pilotage 166 commandant le fonctionnement de la vanne 156, des mélangeurs 155, 159, 162 et des moyens configurés pour produire un souffle 165.

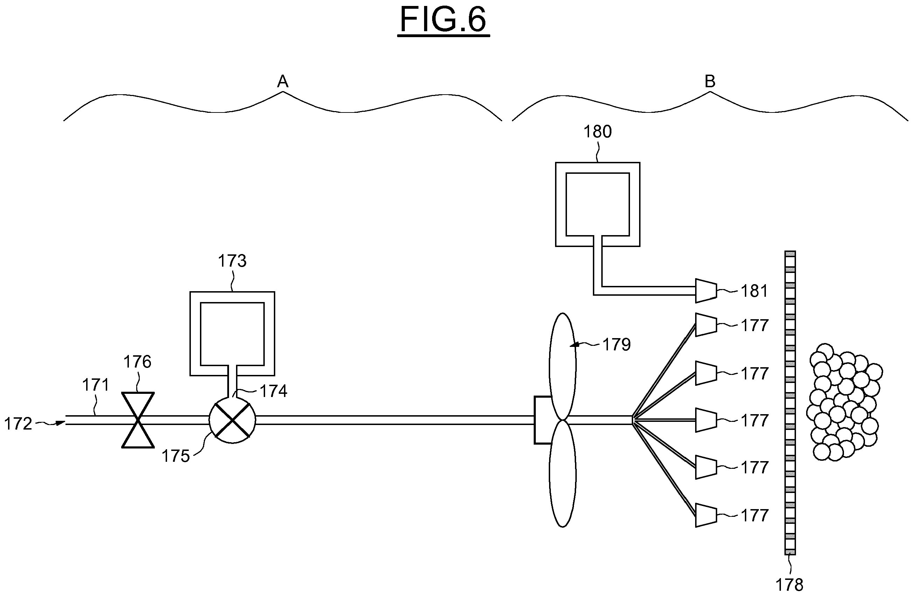

La figure 6 présente un troisième mode de mise en oeuvre de l'installation de production de mousse selon l'invention.

Cette installation comprend une conduite principale 171 ayant une entrée principale 172. Cette entrée principale est reliée à une arrivée de liquide principal. L'arrivée de liquide principal pouvant être reliée de la même manière que décrit précédemment.

L'installation comprend un premier réservoir 173 qui comprend le au moins un agent tensioactif tel que défini précédemment.

Le premier réservoir 173 est en communication fluidique avec une première entrée secondaire 174 de la conduite principale 171.

La communication fluidique s'effectue au moyen d'un mélangeur 175.

De manière préférée, le mélangeur 175 fonctionne avec un système de succion par effet Venturi ou par une pompe d'injection.

En aval de ce premier mélangeur 175, une vanne 176 est présente sur la conduite principale 171.

La conduite principale 171 est reliée à au moins une buse principale 177.

Une grille 178 se situe en aval de ladite au moins une buse principale 177.

Les moyens configurés pour produire un souffle 179 se situe en amont de ladite au moins une buse principale 177 afin de produire un souffle dans le sens de l'écoulement du mélange présent à l'entrée principale du second module. Dans cette figure 6, les moyens pour produire un souffle comprennent un ventilateur.

Le second module comprend en outre un dispositif d'addition du au moins un produit actif qui comprend un réservoir supplémentaire 180 qui comprend le au moins un produit actif principal, ledit réservoir supplémentaire 180 étant relié à au moins une buse supplémentaire 181, l'écoulement du au moins un produit actif étant dans le même sens que l'écoulement du mélange présent à l'entrée du second module.

Pour faire fonctionner l'installation selon la figure 6, l'entrée principale 172 est reliée à une arrivée de liquide principal. Ce liquide principal passe ensuite à travers la vanne 176 pour atteindre le premier mélangeur 175. Le liquide principal est ainsi mélangé à le au moins un agent tensioactif présent dans le premier réservoir 173 et relié à la conduite principale 171 au moyen de la première entrée secondaire 174.

Le mélange ainsi obtenu est projeté au moyen d'au moins une buse principale 177 sur une grille 178. Des moyens configurés pour produire un souffle 179 produisent les bulles de la mousse en projetant du gaz dans le mélange présent sur la grille 178.

A ce mélange est ajouté au moyen de la buse supplémentaire 181, le au moins un produit actif principal stocké dans le réservoir supplémentaire 180.

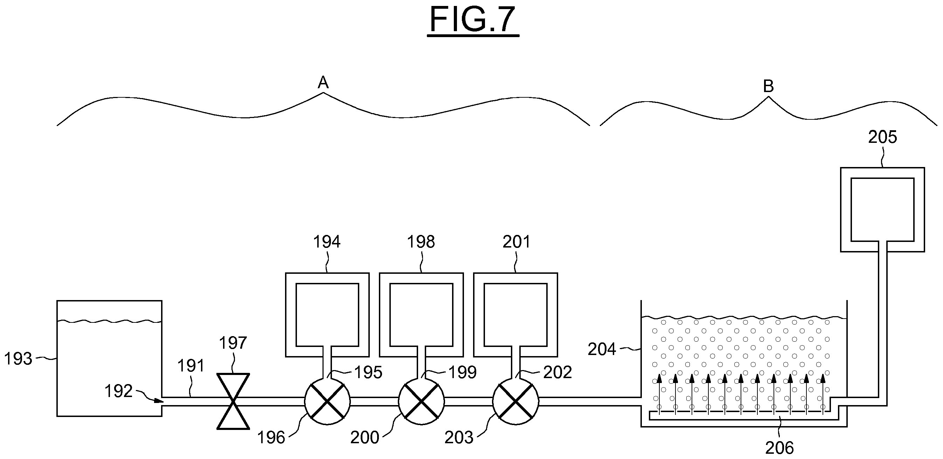

La figure 7 présente un quatrième mode de mise en oeuvre de l'installation de production de mousse selon l'invention.

Cette installation comprend un réservoir principal 193 qui comprend le liquide principal tel que défini précédemment.

Ce réservoir 193 peut notamment être sous pression supérieure à la pression atmosphérique.

Ce réservoir 193 est relié à l'entrée 192 d'une conduite principale 191.

L'installation comprend un premier réservoir 194 qui comprend le au moins un agent tensioactif tel que défini précédemment.

Le premier réservoir 194 est en communication fluidique avec une première entrée secondaire 195 de la conduite principale 191.

La communication fluidique s'effectue au moyen d'un mélangeur 196.

De manière préférée, le mélangeur 196 fonctionne avec un système de succion par effet Venturi ou par une pompe d'injection.

En aval de ce premier mélangeur 196, une vanne 197 est présente sur la conduite principale 191.

Cette installation comprend un deuxième réservoir 198 qui comprend le au moins un produit actif principal tel que défini précédemment.

Le deuxième réservoir 198 est en communication fluidique avec une deuxième entrée secondaire 199 de la conduite principale 191. Cette deuxième entrée secondaire 199 se situe en aval de la première entrée secondaire 195.

La communication fluidique s'effectue au moyen d'un mélangeur 200.

De manière préférée, le mélangeur 200 fonctionne avec un système de succion par effet Venturi ou par une pompe d'injection.

Cette installation selon la figure 7 comprend en outre un troisième réservoir 201 qui comprend le au moins un additif tel que défini précédemment.

Le troisième réservoir 201 étant en communication fluidique avec une troisième entrée secondaire 202 de la conduite principale 191. Cette troisième entrée secondaire 202 se situe en aval de la deuxième entrée secondaire 199.

La communication fluidique s'effectue au moyen d'un mélangeur 203.

De manière préférée, le mélangeur 203 fonctionne avec un système de succion par effet Venturi ou par une pompe d'injection.

La conduite principale 191 est reliée à un réservoir final 204. Ce réservoir final 204 comprend un dispositif de barbotage 206. Ce dispositif de barbotage est alimenté au moyen d'un réservoir 205 sous pression supérieure à la pression atmosphérique.

Pour faire fonctionner l'installation selon la figure 7, le réservoir principal 193 est rempli en liquide principal, le tout étant sous une pression supérieure à la pression atmosphérique.

Le liquide principal s'écoule à travers l'entrée principale 192 de la conduite principale 191. Ce liquide principal passe ensuite à travers la vanne 197 pour atteindre le premier mélangeur 196. Le liquide principal est ainsi mélangé à le au moins un agent tensioactif présent dans le premier réservoir 194 et relié à la conduite principale 191 au moyen de la première entrée secondaire 195.

Le mélange ainsi obtenu arrive dans le deuxième mélangeur 200. Le mélange est ainsi brassé avec le au moins un produit actif principal présent dans le deuxième réservoir 198 et relié à la conduite principale 191 au moyen de la deuxième entrée secondaire 199.

Le mélange ainsi obtenu arrive dans le troisième mélangeur 203. Le mélange est brassé avec le au moins un additif présent dans le troisième réservoir 201 et relié à la conduite principale 191 au moyen de la troisième entrée secondaire 202.

Le mélange ainsi obtenu est stocké dans un réservoir final 204. Dans ce réservoir final 204, un gaz est mis à barboter au moyen d'un système de barbotage 206. Le système de barbotage 206 permet de produire la mousse. Ce système de barbotage 206 est alimenté au moyen d'un réservoir 205 qui comprend le gaz sous pression supérieure à la pression atmosphérique.

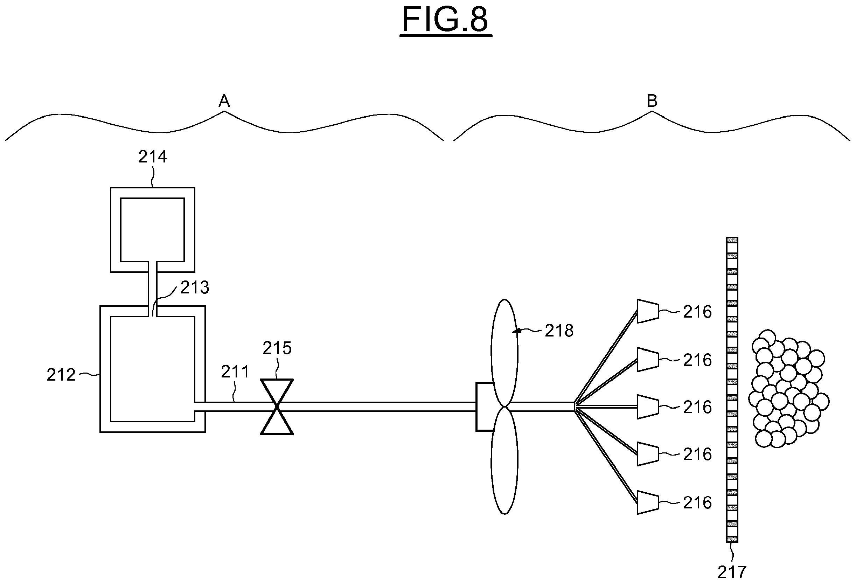

La figure 8 présente un cinquième mode de mise en oeuvre de l'installation de production de mousse selon l'invention.

Cette installation comprend une conduite principale 211. Cette conduite principale est reliée d'une part à un réservoir unique 212 qui comprend le liquide principal, le au moins un agent tensioactif et le au moins produit actif principal.

Ce réservoir unique comprend une entrée 213 reliée à un système pyrotechnique ou à un système de stockage et/ou de production de gaz comprimé 214.

Cette conduite principale comprend en outre une vanne 215 et est reliée d'autre part à au moins une buse principale 216.

Une grille 217 se situe en aval de ladite au moins une buse principale 216.

Les moyens configurés pour produire un souffle 218 se situent en amont de ladite au moins une buse principale 216 afin de produire un souffle dans le sens de l'écoulement du mélange présent à l'entrée principale du second module. Dans cette figure 8, les moyens pour produire un souffle comprennent un ventilateur.

Pour faire fonctionner l'installation selon la figure 8, le réservoir unique 212 comprend un mélange principal qui comprend le liquide principal, le au moins un agent tensioactif et le au moins un produit actif principal. Ce mélange principal est poussé vers la conduite 211 au moyen du système pyrotechnique ou du système de stockage et/ou de production de gaz comprimé 214 relié à l'entrée 213 du réservoir unique 212.

Après être passé à travers la vanne 215, le mélange principal est projeté au moyen d'au moins une buse principale 216 sur une grille 217. Des moyens configurés pour produire un souffle 218 produisent les bulles de la mousse en projetant du gaz dans le mélange présent sur la grille 217.

## Revendications

1. Installation de production de mousse présentant un coefficient de foisonnement supérieur à 10, comprenant :
- un premier module (A) comprenant une sortie principale et délivrant à ladite sortie principale un mélange comprenant un liquide principal et au moins un agent tensioactif,
- un second module (B) couplé à la sortie principale du premier module (A) et produisant une mousse qui comprend au moins le liquide principal, le au moins un agent tensioactif et au moins un produit actif principal, à partir du mélange délivré par la sortie principale du premier module (A),
le mélange comprenant le au moins un produit actif principal ou
le second module (B) comprenant en outre des moyens configurés pour que la mousse produite par le second module (B) comprenne le au moins un produit actif principal, **caractérisée en ce que** ledit au moins un produit actif principal est choisi parmi les produits irritants, les produits annihilant les capacités musculaires et/ou de réflexion d'un être vivant,
la mousse produite par le second module comprenant en outre au moins un additif augmentant la viscosité,
l'additif augmentent la viscosité étant une gomme arabique, le ou les produits irritants et le ou les produits annihilant les capacités musculaires et/ou de réflexion d'un être vivant étant encapsulés dans la gomme arabique,

2. Installation selon la revendication 1, **caractérisée en ce que** la mousse produite par l'installation présente un coefficient de foisonnement supérieur à 20, de préférence variant de 20 à 2000, plus préférentiellement variant de 40 à 2000, encore plus préférentiellement variant de 60 à 2000, encore plus préférentiellement variant de 150 à 2000, et en particulier variant de 400 à 2000.

3. Installation selon la revendication 1 ou 2, **caractérisée en ce que** le mélange délivré par la sortie principale du premier module comprend le au moins un produit actif principal et ce premier module comprend :
- soit un réservoir unique (1) qui comprend le liquide principal, le au moins un agent tensioactif et le au moins un produit actif principal, ce réservoir étant relié à la sortie principale (2) du premier module,
- soit un réservoir principal (11) qui comprend le liquide principal, ledit réservoir principal (11) étant relié à un entrée principale (13) d'une conduite principale (14), et un premier réservoir (15) qui comprend le au moins un agent tensioactif et le au moins un produit actif principal, ledit premier réservoir (15) étant en communication fluidique (16) avec une première entrée secondaire (17) de la conduite principale (14), ladite conduite principale (14) étant reliée à la sortie principale (12) du premier module,
- soit un réservoir principal (11) qui comprend le liquide principal et le au moins un agent tensioactif, ledit réservoir principal (11) étant relié à un entrée principale (13) d'une conduite principale (14), et un premier réservoir (15) qui comprend le au moins un produit actif principal, ledit premier réservoir (15) étant en communication fluidique (16) avec une première entrée secondaire (17) de la conduite principale (14), ladite conduite principale (14) étant reliée à la sortie principale (12) du premier module,
- soit un réservoir principal (11) qui comprend le liquide principal et le au moins un produit actif principal, ledit réservoir principal (11) étant relié à un entrée principale (13) d'une conduite principale (14), et un premier réservoir (15) qui comprend le au moins un agent tensioactif, ledit premier réservoir (15) étant en communication fluidique (16) avec une première entrée secondaire (17) de la conduite principale (14), ladite conduite principale (14) étant reliée à la sortie principale (12) du premier module,
- soit un réservoir principal (21) qui comprend le liquide principal, ledit réservoir principal (21) étant relié à une entrée principale (23) d'une conduite principale (24), un premier réservoir (25) qui comprend le au moins un agent tensioactif, ledit premier réservoir (25) étant en communication fluidique (26) avec une première entrée secondaire (27) de la conduite principale (24), et un deuxième réservoir (28) qui comprend le au moins un produit actif principal, ledit deuxième réservoir (28) étant en communication fluidique (29) avec une deuxième entrée secondaire (30) de la conduite principale (24), ladite conduite principale (24) étant reliée à la sortie principale (22) du premier module,
- soit une entrée principale (33) d'une conduite principale (34) reliée à une arrivée du liquide principal, un premier réservoir (35) qui comprend le au moins un agent tensioactif et le au moins un produit actif principal, ledit premier réservoir (35) étant en communication fluidique (36) avec une première entrée secondaire (37) de la conduite principale (34), ladite conduite principale (34) étant reliée à la sortie principale (32) du premier module,
- soit une entrée principale (43) d'une conduite principale (44) reliée à une arrivée du liquide principal, un premier réservoir (45) qui comprend le au moins un agent tensioactif, ledit premier réservoir (45) étant en communication fluidique (46) avec une première entrée secondaire (47) de la conduite principale (44), et un deuxième réservoir (48) qui comprend le au moins un produit actif principal, ledit deuxième réservoir (48) étant en communication fluidique (49) avec une deuxième entrée secondaire (50) de la conduite principale (44), ladite conduite principale (44) étant reliée à la sortie principale (42) du premier module.

4. Installation selon la revendication 1 ou 2, **caractérisée en ce que** le second module comprend en outre des moyens configurés pour que la mousse produite par le second module comprenne le au moins un produit actif principal et le premier module comprend :
- soit un réservoir unique (51) qui comprend le liquide principal, le au moins un agent tensioactif et optionnellement au moins un produit actif secondaire, ce réservoir (51) étant relié à la sortie principale (52) du premier module,
- soit un réservoir principal (61) qui comprend le liquide principal, et optionnellement au moins un produit actif secondaire, ledit réservoir principal (61) étant relié à une entrée principale (63) d'une conduite principale (64), et un premier réservoir (65) qui comprend le au moins un agent tensioactif, ledit premier réservoir (65) étant en communication fluidique (66) avec une première entrée secondaire (67) de la conduite principale (64), ladite conduite principale (64) étant reliée à la sortie principale (62) du premier module,
- soit un réservoir principal (61) qui comprend le liquide principal, ledit réservoir principal (61) étant relié à une entrée principale (63) d'une conduite principale (64), et un premier réservoir (65) qui comprend le au moins un agent tensioactif et optionnellement au moins un produit actif secondaire, ledit premier réservoir (65) étant en communication fluidique (66) avec une première entrée secondaire (67) de la conduite principale (64), ladite conduite principale (64) étant reliée à la sortie principale (62) du premier module,
- soit un réservoir principal (61) qui comprend le liquide principal et le au moins un agent tensioactif, ledit réservoir principal (61) étant relié à une entrée principale (63) d'une conduite principale (64), et un premier réservoir (65) qui comprend au moins un produit actif secondaire, ledit premier réservoir (65) étant en communication fluidique (66) avec une première entrée secondaire (67) de la conduite principale (64), ladite conduite principale (64) étant reliée à la sortie principale (62) du premier module,
- soit un réservoir principal (71) qui comprend le liquide principal, ledit réservoir principal (71) étant relié à une entrée principale (73) d'une conduite principale (74), un premier réservoir (75) qui comprend le au moins un agent tensioactif, ledit premier réservoir (75) étant en communication fluidique (76) avec une première entrée secondaire (77) de la conduite principale (74), et un deuxième réservoir (78) qui comprend au moins un produit actif secondaire, ledit deuxième réservoir (78) étant en communication fluidique (79) avec une deuxième entrée secondaire (80) de la conduite principale (74), ladite conduite principale (74) étant reliée à la sortie principale (72) du premier module,
- soit une entrée principale (83) d'une conduite principale (84) reliée à une arrivée du liquide principal, un premier réservoir (85) qui comprend le au moins un agent tensioactif et optionnellement au moins un produit actif secondaire, ledit premier réservoir (85) étant en communication fluidique (86) avec une première entrée secondaire (87) de la conduite principale (84), ladite conduite principale (84) étant reliée à la sortie principale (82) du premier module,
- soit une entrée principale (93) d'une conduite principale (94) reliée à une arrivée du liquide principal, un premier réservoir (95) qui comprend le au moins un agent tensioactif, ledit premier réservoir (95) étant en communication fluidique (96) avec une première entrée secondaire (97) de la conduite principale (94), et un deuxième réservoir (98) qui comprend au moins un produit actif secondaire, ledit deuxième réservoir (98) étant en communication fluidique (99) avec une deuxième entrée secondaire (100) de la conduite principale (94), ladite conduite principale (94) étant reliée à la sortie principale (92) du premier module.

5. Installation selon l'une quelconque des revendications précédentes, **caractérisée en ce que** le second module comprend une entrée principale (101, 111, 121) reliée à la sortie principale du premier module, ladite entrée principale (101, 111, 121) étant reliée :
- soit à au moins une buse principale (102), une grille (103) étant disposée en aval de ladite au moins une buse principale (102), et, en amont de ladite au moins une buse principale (102), des moyens configurés pour produire un souffle (104) dans le sens de l'écoulement du mélange présent à l'entrée principale (101, 111, 121) du second module,
- soit à au moins une buse principale (112), une section d'admission (114) de gaz en forme de carter étant disposée en aval de ladite au moins une buse principale (112), l'axe longitudinal de cette section d'admission (114) étant parallèle ou identique à l'axe de projection de ladite au moins une buse principale (112), et en aval de cette section d'admission (114), une grille (113) disposée sur la sortie de la section d'admission (114),
- soit à un réservoir final (122), ledit réservoir final comprenant un système de barbotage (123).

6. Installation selon l'une quelconque des revendications 2 à 5, **caractérisée en ce que** le réservoir unique du premier module ou le réservoir principal du premier module ont une entrée reliée à un système pyrotechnique, ou à un système de stockage et/ou de production de gaz comprimé (214).

7. Installation selon la revendication 6, **caractérisée en ce que** le système de stockage et/ou de production de gaz comprimé comprend un compresseur et/ou une bouteille de gaz sous pression.

8. Installation selon l'une quelconque des revendications précédentes, **caractérisée en ce que** le premier module comprend au moins un troisième réservoir (140, 160, 201) qui comprend le au moins un additif, ledit troisième réservoir (140, 160, 201) étant en communication fluidique avec une troisième entrée secondaire (141, 161, 202) de la conduite principale du premier module.

9. Installation selon l'une quelconque des revendications précédentes, **caractérisée en ce que** le mélange délivré en sortie principale du premier module a une teneur en additif variant de 0,0001 à 10 % en poids, de préférence de 0,001 % à 1 % en poids, par rapport au poids total du mélange délivré en sortie principale du premier module.

10. Installation selon l'une quelconque des revendications 1 à 2 et 4 à 9, **caractérisée en ce que** les moyens configurés pour que la mousse produite par le second module comprenne le au moins un produit actif principal, comprennent un dispositif d'addition qui comprend un réservoir supplémentaire (180) qui comprend le au moins un produit actif principal, le réservoir supplémentaire (180) étant relié à au moins un moyen apte à ajouter le au moins un produit actif principal au mélange présent à l'entrée principale du second module.

11. Installation selon la revendication 10, **caractérisée en ce que**, lorsque l'entrée principale du second module est reliée à au moins une buse principale, le moyen apte à ajouter le au moins un produit actif principal au mélange présent à l'entrée principale du second module comprend au moins une buse supplémentaire (181), l'écoulement du au moins un produit actif principal étant dans le même sens que l'écoulement du mélange présent à l'entrée principale du second module.

12. Installation selon l'une quelconque des revendications 4 à 11, **caractérisée en ce que** ledit au moins un produit actif secondaire est choisi parmi les colorants, les produits fluorescents, les marqueurs chimiques et/ou biologiques, les produits irritants, les gaz, les produits annihilant les capacités musculaires et/ou de réflexion d'un être vivant et les réflecteurs de radar.

13. Installation selon l'une quelconque des revendications 1 à 12, **caractérisée en ce que** ledit au moins un produit actif principal et ledit au moins un produit actif secondaire éventuel sont choisis parmi les produits irritants et les produits annihilant les capacités musculaires et/ou de réflexion d'un être vivant, et plus particulièrement ledit au moins un produit actif principal et ledit au moins un produit actif secondaire éventuel sont la capsaïcine, l'oléorésine de capsicum ou un de ses dérivés et le nonivamide.

14. Véhicule automobile, navire ou aéronef comprenant l'installation telle que définie dans l'une quelconque des revendications 1 à 13.

15. Procédé de production de mousse comprenant les étapes suivantes :
i) un premier module qui délivre en sortie principale un mélange comprenant un liquide principal et au moins un agent tensioactif, puis
ii) un deuxième module relié à la sortie principale du premier module qui produit une mousse qui comprend le liquide principal, le au moins un agent tensioactif et au moins un produit actif principal, la mousse produite présentant un coefficient de foisonnement supérieur à 10, avantageusement supérieur à 20, de préférence variant de 20 à 2000, plus préférentiellement variant de 40 à 2000, encore plus préférentiellement variant de 60 à 2000, encore plus préférentiellement variant de 150 à 2000, et en particulier variant de 400 à 2000, **caractérisé en ce que** ledit au moins un produit actif principal est choisi parmi les produits irritants, les produits annihilant les capacités musculaires et/ou de réflexion d'un être vivant,
la mousse produite par le second module comprenant en outre au moins un additif augmentant la viscosité, l'additif augmentant la viscosité étant une gomme arabique, le ou les produits irritants et le ou les produits annihilant les capacités musculaires et/ou de réflexion d'un être vivant étant encapsulés dans la gomme arabique.

## Patentansprüche

1. Ausrüstung zur Schaumstoffherstellung, der einen Expansionsfaktor größer als 10 aufweist, Folgendes umfassend:
- ein erstes Modul (A), das einen Hauptausgang umfasst und an dem Hauptausgang ein Gemisch ausgibt, das eine Hauptflüssigkeit und mindestens ein oberflächenaktives Mittel umfasst,
- ein zweites Modul (B), das an den Hauptausgang des ersten Moduls (A) gekoppelt ist, und einen Schaumstoff, der mindestens die Hauptflüssigkeit, das mindestens eine oberflächenaktive Mittel, und mindestens einen Hauptwirkstoff umfasst, aus dem Gemisch herstellt, das durch den Hauptausgang des ersten Moduls (A) ausgegeben wird,
wobei das Gemisch den mindestens einen Hauptwirkstoff umfasst oder
das zweite Modul (B) weiter Mittel umfasst, die konfiguriert sind, damit der durch das zweite Modul (B) herstellte Schaumstoff den mindestens einen Hauptwirkstoff umfasst, **dadurch gekennzeichnet, dass** der mindestens eine Hauptwirkstoff ausgewählt ist aus den reizerzeugenden Stoffen, den Stoffen, die das Muskel- und/ oder Denkvermögen eines Lebewesens,
wobei der durch das zweite Modul hergestellte Schaumstoff weiter mindestens einen die Viskosität erhöhenden Zusatzstoff umfasst, wobei der die Viskosität erhöhende Zusatzstoff ein Gummiarabikum ist, das oder die reizerzeugenden Stoffe und das oder die Stoffe, die das Muskel- und/ oder Denkvermögen eines Lebewesens unterdrücken, in dem Gummiarabikum eingekapselt sind.

2. Ausrüstung nach Anspruch 1, **dadurch gekennzeichnet, dass** der durch die Ausrüstung hergestellte Schaumstoff einen Expansionsfaktor von mehr als 20, vorzugsweise von 20 bis 2000 variierend, weiter bevorzugt von 40 bis 2000 variierend, noch bevorzugter von 60 bis 2000 variierend, noch bevorzugter von 150 bis 2000 variierend, und insbesondere von 400 bis 2000 variierend aufweist.

3. Ausrüstung nach Anspruch 1 oder 2, **dadurch gekennzeichnet, dass** das durch den Hauptausgang des ersten Moduls ausgegebene Gemisch den mindestens einen Hauptwirkstoff umfasst, und dieses erste Modul umfasst:
- entweder einen einzigen Behälter (1), der die Hauptflüssigkeit, das mindestens eine oberflächenaktive Mittel, und den mindestens einen Hauptwirkstoff umfasst, wobei dieser Behälter an den Hauptausgang (2) des ersten Moduls angeschlossen ist,
- oder einen Hauptbehälter (11), der die Hauptflüssigkeit umfasst, wobei der Hauptbehälter (11) an einen Haupteingang (13) einer Hauptleitung (14) angeschlossen ist, und einen ersten Behälter (15), der das mindestens eine oberflächenaktive Mittel, und den mindestens einen Hauptwirkstoff umfasst, wobei der erste Behälter (15) in Fluidverbindung (16) mit einem ersten sekundären Eingang (17) der Hauptleitung (14) ist, wobei die Hauptleitung (14) an den Hauptausgang (12) des ersten Moduls angeschlossen ist,
- oder einen Hauptbehälter (11), der die Hauptflüssigkeit und das mindestens eine oberflächenaktive Mittel umfasst, wobei der Hauptbehälter (11) an einen Haupteingang (13) einer Hauptleitung (14) angeschlossen ist, und einen ersten Behälter (15), der den mindestens einen Hauptwirkstoff umfasst, wobei der erste Behälter (15) in Fluidverbindung (16) mit einem ersten sekundären Eingang (17) der Hauptleitung (14) ist, wobei die Hauptleitung (14) an den Hauptausgang (12) des ersten Moduls angeschlossen ist,
- oder einen Hauptbehälter (11), der die Hauptflüssigkeit und den mindestens einen Hauptwirkstoff umfasst, wobei der Hauptbehälter (11) an einen Haupteingang (13) einer Hauptleitung (14) angeschlossen ist, und einen ersten Behälter (15), der das mindestens eine oberflächenaktive Mittel umfasst, wobei der erste Behälter (15) in Fluidverbindung (16) mit einem ersten sekundären Eingang (17) der Hauptleitung (14) ist, wobei die Hauptleitung (14) an den Hauptausgang (12) des ersten Moduls angeschlossen ist,
- oder einen Hauptbehälter (21), der die Hauptflüssigkeit umfasst, wobei der Hauptbehälter (21) an einen Haupteingang (23) einer Hauptleitung (24) angeschlossen ist, einen ersten Behälter (25), der das mindestens eine oberflächenaktive Mittel umfasst, wobei der erste Behälter (25) in Fluidverbindung (26) mit einem ersten sekundären Eingang (27) der Hauptleitung (24) ist, und einen zweiten Behälter (28), der den mindestens einen Hauptwirkstoff umfasst, wobei der zweite Behälter (28) in Fluidverbindung (29) mit einem zweiten sekundären Eingang (30) der Hauptleitung (24) ist, wobei die Hauptleitung (24) an den Hauptausgang (22) des ersten Moduls angeschlossen ist,
- oder einen Haupteingang (33) einer Hauptleitung (34), die an eine Zuführung der Hauptflüssigkeit angeschlossen ist, einen ersten Behälter (35), der das mindestens eine oberflächenaktive Mittel und den mindestens einen Hauptwirkstoff umfasst, wobei der erste Behälter (35) in Fluidverbindung (36) mit einem ersten sekundären Eingang (37) der Hauptleitung (34) ist, wobei die Hauptleitung (34) an den Hauptausgang (32) des ersten Moduls angeschlossen ist,
- oder einen Haupteingang (43) einer Hauptleitung (44), die an eine Zuführung der Hauptflüssigkeit angeschlossen ist, einen ersten Behälter (45), der das mindestens eine oberflächenaktive Mittel umfasst, wobei der erste Behälter (45) in Fluidverbindung (46) mit einem ersten sekundären Eingang (47) der Hauptleitung (44) ist, und einen zweiten Behälter 48), der den mindestens einen Hauptwirkstoff umfasst, wobei der zweite Behälter (48) in Fluidverbindung (49) mit einem zweiten sekundären Eingang (50) der Hauptleitung (44) ist, wobei die Hauptleitung (44) an den Hauptausgang (42) des ersten Moduls angeschlossen ist.

4. Ausrüstung nach Anspruch 1 oder 2, **dadurch gekennzeichnet, dass** das zweite Modul weiter Mittel umfasst, die konfiguriert sind, damit der von dem zweiten Modul hergestellte Schaumstoff den mindestens einen Hauptwirkstoff umfasst, und das erste Modul Folgendes umfasst:
- entweder einen einzigen Behälter (51), der die Hauptflüssigkeit, das mindestens eine oberflächenaktive Mittel, und gegebenenfalls mindestens einen sekundären Wirkstoff umfasst, wobei dieser Behälter (51) an den Hauptausgang (52) des ersten Moduls angeschlossen ist,
- oder einen Hauptbehälter (61), der die Hauptflüssigkeit, und gegebenenfalls mindestens einen sekundären Wirkstoff umfasst, wobei der Hauptbehälter (61) an einen Haupteingang (63) einer Hauptleitung (64) angeschlossen ist, und einen ersten Behälter (65), der das mindestens eine oberflächenaktive Mittel umfasst, wobei der erste Behälter (65) in Fluidverbindung (66) mit einem ersten sekundären Eingang (67) der Hauptleitung (64) ist, wobei die Hauptleitung (64) an den Hauptausgang (62) des ersten Moduls angeschlossen ist,
- oder einen Hauptbehälter (61), der die Hauptflüssigkeit umfasst, wobei der Hauptbehälter (61) an einen Haupteingang (63) einer Hauptleitung (64) angeschlossen ist, und einen ersten Behälter (65), der das mindestens eine oberflächenaktive Mittel, und gegebenenfalls mindestens einen sekundären Wirkstoff umfasst, wobei der erste Behälter (65) in Fluidverbindung (66) mit einem ersten sekundären Eingang (67) der Hauptleitung (64) ist, wobei die Hauptleitung (64) an den Hauptausgang (62) des ersten Moduls angeschlossen ist,
- oder einen Hauptbehälter (61), der die Hauptflüssigkeit und das mindestens eine oberflächenaktive Mittel umfasst, wobei der Hauptbehälter (61) an einen Haupteingang (63) einer Hauptleitung (64) angeschlossen ist, und einen ersten Behälter (65), der das mindestens einen sekundären Wirkstoff umfasst, wobei der erste Behälter (65) in Fluidverbindung (66) mit einem ersten sekundären Eingang (67) der Hauptleitung (64) ist, wobei die Hauptleitung (64) an den Hauptausgang (62) des ersten Moduls angeschlossen ist,
- oder einen Hauptbehälter (71), der die Hauptflüssigkeit umfasst, wobei der Hauptbehälter (71) an einen Haupteingang (73) einer Hauptleitung (74) angeschlossen ist, einen ersten Behälter (75), der das mindestens eine oberflächenaktive Mittel umfasst, wobei der erste Behälter (75) in Fluidverbindung (76) mit einem ersten sekundären Eingang (77) der Hauptleitung (74) ist, und einen zweiten Behälter (78), der mindestens einen sekundären Wirkstoff umfasst, wobei der zweite Behälter (78) in Fluidverbindung (79) mit einem zweiten sekundären Eingang (80) der Hauptleitung (74) ist, wobei die Hauptleitung (74) an den Hauptausgang (72) des ersten Moduls angeschlossen ist,
- oder einen Haupteingang (83) einer Hauptleitung (84), die an eine Zuführung der Hauptflüssigkeit angeschlossen ist, einen ersten Behälter (85), der das mindestens eine oberflächenaktive Mittel, und gegebenenfalls mindestens einen sekundären Wirkstoff umfasst, wobei der erste Behälter (85) in Fluidverbindung (86) mit einem ersten sekundären Eingang (87) der Hauptleitung (84) ist, wobei die Hauptleitung (84) an den Hauptausgang (82) des ersten Moduls angeschlossen ist,
- oder einen Haupteingang (93) einer Hauptleitung (94), die an eine Zuführung der Hauptflüssigkeit angeschlossen ist, einen ersten Behälter (95), der das mindestens eine oberflächenaktive Mittel umfasst, wobei der erste Behälter (95) in Fluidverbindung (96) mit einem ersten sekundären Eingang (97) der Hauptleitung (94) ist, und einen zweiten Behälter (98), der mindestens einen sekundären Wirkstoff umfasst, wobei der zweite Behälter (98) in Fluidverbindung (99) mit einem zweiten sekundären Eingang (100) der Hauptleitung (94) ist, wobei die Hauptleitung (94) an den Hauptausgang (92) des ersten Moduls angeschlossen ist.

5. Ausrüstung nach einem der vorstehenden Ansprüche, **dadurch gekennzeichnet, dass** das zweite Modul einen Haupteingang (101, 111, 121) umfasst, der an den Hauptausgang des ersten Moduls angeschlossen ist, wobei der Haupteingang (101, 111, 121) angeschlossen ist:
- entweder an mindestens eine Hauptdüse (102), wobei ein Gitter (103) stromabwärts der mindestens einen Hauptdüse (102) angeordnet ist, und stromaufwärts der mindestens einen Hauptdüse (102), Mittel, die konfiguriert sind, um einen Blasimpuls (104) in der Richtung der Strömung des Gemisches zu erzeugen, das am Haupteingang (101, 111, 121) des zweiten Moduls vorhanden ist,
- oder an mindestens eine Hauptdüse (112), wobei eine Gaseinlasssektion (114) in Form eines Gehäuses stromabwärts der mindestens einen Hauptdüse (112) angeordnet ist, wobei die Längsachse dieser Einlasssektion (114) parallel oder identisch zur Projektionsachse der mindestens einen Hauptdüse (112) ist, und stromabwärts dieser Einlasssektion (114) ein Gitter (113), das am Ausgang der Einlasssektion (114) angeordnet ist,
- oder an einen Endbehälter (122), wobei dieser Endbehälter ein Tauchsystem (123) umfasst.

6. Ausrüstung nach einem der Ansprüche 2 bis 5, **dadurch gekennzeichnet, dass** der einzige Behälter des ersten Moduls oder der Hauptbehälter des ersten Moduls einen Eingang aufweisen, der an ein pyrotechnisches System oder ein System zum Lagern und/ oder Erzeugen von komprimiertem Gas (214) angeschlossen ist.

7. Ausrüstung nach Anspruch 6, **dadurch gekennzeichnet, dass** das System zum Lagern und/ oder Erzeugen von komprimiertem Gas einen Kompressor und/ oder eine Druckgasflasche umfasst.

8. Ausrüstung nach einem der vorstehenden Ansprüche, **dadurch gekennzeichnet, dass** das erste Modul mindestens einen dritten Behälter (140, 160, 201) umfasst, der den mindestens einen Zusatzstoff umfasst, wobei der dritte Behälter (140, 160, 201) in Fluidverbindung mit einem dritten sekundären Eingang (141, 161, 202) der Hauptleitung des ersten Moduls ist.

9. Ausrüstung nach einem der vorstehenden Ansprüche, **dadurch gekennzeichnet, dass** das am Hauptausgang des ersten Moduls ausgegebene Gemisch einen Gehalt an Zusatzstoff aufweist, der von 0,0001 bis 10 Gew.-%, vorzugsweise von 0,001 Gew.-% bis 1 Gew.-% in Bezug auf das Gesamtgewicht des am Hauptausgang des ersten Moduls ausgegebenen Gemisches variiert.

10. Ausrüstung nach einem der Ansprüche 1 bis 2 und 4 bis 9, **dadurch gekennzeichnet, dass** die Mittel, die konfiguriert sind, damit das durch das zweite Modul herstellte Produkt den mindestens einen Hauptwirkstoff umfasst, eine Vorrichtung zum Hinzufügen umfassen, die einen zusätzlichen Behälter (180) umfasst der den mindestens einen Hauptwirkstoff umfasst, wobei der zusätzliche Behälter (180) an mindestens ein Mittel angeschlossen ist, das imstande ist, den mindestens einen Hauptwirkstoff zum Gemisch hinzuzufügen, das am Haupteingang des zweiten Moduls vorhanden ist.

11. Ausrüstung nach Anspruch 10, **dadurch gekennzeichnet, dass**, wenn der Haupteingang des zweiten Moduls an mindestens eine Hauptdüse angeschlossen ist, das Mittel, das imstande ist, den mindestens einen Hauptwirkstoff zum Gemisch hinzuzufügen, das am Haupteingang des zweiten Moduls vorhanden ist, mindestens eine zusätzliche Düse (181) umfasst, wobei die Strömung des mindestens einen Hauptwirkstoffes in derselben Richtung erfolgt, wie die Strömung des Gemisches, das am Haupteingang des zweiten Moduls vorhanden ist.

12. Ausrüstung nach einem der Ansprüche 4 bis 11, **dadurch gekennzeichnet, dass** der mindestens eine sekundäre Wirkstoff aus den Farbstoffen, den fluoreszierenden Stoffen, den chemischen und/ oder biologischen Markern, den reizerzeugenden Stoffen, den Gasen, den Stoffen, die das Muskel- und/ oder Denkvermögen eines Lebewesens und die Radarreflektoren unterdrücken, ausgewählt ist.

13. Ausrüstung nach einem der Ansprüche 1 bis 12, **dadurch gekennzeichnet, dass** der mindestens eine Hauptwirkstoff und der eventuelle mindestens eine sekundäre Wirkstoff aus den reizerzeugenden Stoffen und den Stoffen, die das Muskel- und/ oder Denkvermögen eines Lebewesens unterdrücken, ausgewählt sind, und insbesondere der mindestens eine Hauptwirkstoff und der mindestens eine eventuelle sekundäre Wirkstoff Capsaicin, Oleoresin Capsicum, oder eines ihrer Derivate, oder Nonivamid sind.

14. Kraftfahrzeug, Schiff oder Luftfahrzeug, die Ausrüstung nach einem der Ansprüche 1 bis 13 umfassend.

15. Verfahren zur Herstellung von Schaumstoff, die folgenden Schritte umfassend:
i) Ausgabe am Hauptausgang eines ersten Moduls eines Gemisches, das eine Hauptflüssigkeit und mindestens ein oberflächenaktives Mittel umfasst, danach
ii) Herstellen, in einem zweiten Modul, das an den Hauptausgang des ersten Moduls angeschlossen ist, eines Schamstoffes, der die Hauptflüssigkeit, das mindestens eine oberflächenaktive Mittel und mindestens einen Hauptwirkstoff umfasst, wobei der hergestellte Schaumstoff einen Expansionsfaktor von mehr als 10, vorteilhaft von mehr als 20, vorzugsweise von 20 bis 2000 variierend, weiter bevorzugt von 40 bis 2000 variierend, und noch bevorzugter von 60 bis 2000 variierend, noch bevorzugter von 150 bis 2000 variierend, und insbesondere von 400 bis 2000 variierend aufweist, **dadurch gekennzeichnet, dass** der mindestens eine Hauptwirkstoff ausgewählt ist aus den reizerzeugenden Stoffen, den Stoffen, die das Muskel- und/ oder Denkvermögen eines Lebewesens unterdrücken,
wobei der durch das zweite Modul hergestellte Schaumstoff weiter mindestens einen die Viskosität erhöhenden Zusatzstoff umfasst, wobei der die Viskosität erhöhende Zusatzstoff ein Gummiarabikum ist, das oder die reizerzeugenden Stoffe und der oder die Stoffe, die das Muskel- und/ oder Denkvermögen eines Lebewesens, in dem Gummiarabikum eingekapselt sind.

## Claims

1. Equipment for producing foam having a swell factor greater than 10, comprising:
- a first module (A) comprising a main outlet and delivering at said main outlet a mixture comprising a main liquid and at least one surfactant,
- a second module (B) coupled to the main outlet of the first module (A) and producing a foam that comprises at least the main liquid, the at least one surfactant and at least one main active product, from the mixture delivered by the main outlet of the first module (A),
the mixture comprising the at least one main active product, or
the second module (B) further comprising means configured so that the foam produced by the second module (B) comprises the at least one main active product, **characterised in that** said at least one main active product is selected from irritant products and products destroying the muscular and/or or thinking capabilities of a living being,
the foam produced by the second module further comprising at least one viscosity-increasing additive,
the viscosity-increasing additive being a gum arabic, the irritant product or products and the product or products destroying the muscular and/or thinking capabilities of a living being being encapsulated in the gum arabic.

2. Equipment according to claim 1, **characterised in that** the foam produced by the equipment has a swell factor greater than 20, preferably varying from 20 to 2000, more preferentially varying from 40 to 2000, even more preferentially varying from 60 to 2000, even more preferentially varying from 150 to 2000, and in particular varying from 400 to 2000.

3. Equipment according to claim 1 or 2, **characterised in that** the mixture delivered by the main outlet of the first module comprises the at least one main active product and this first module comprises:
- either a single reservoir (1) that comprises the main liquid, the at least one surfactant and the at least one main active product, this reservoir being connected to the main outlet (2) of the first module,
- or a main reservoir (11) that comprises the main liquid, said main reservoir (11) being connected to a main inlet (13) of a main conduit (14), and a first reservoir (15) that comprises the at least one surfactant and the at least one main active product, said first reservoir (15) being in fluid communication (16) with a first secondary inlet (17) of the main conduit (14), said main conduit (14) being connected to the main outlet (12) of the first module,
- or a main reservoir (11) that comprises the main liquid and the at least one surfactant, said main reservoir (11) being connected to a main inlet (13) of a main conduit (14), and a first reservoir (15) that comprises the at least one main active product, said first reservoir (15) being in fluid communication (16) with a first secondary inlet (17) of the main conduit (14), said main conduit (14) being connected to the main outlet (12) of the first module,
- or a main reservoir (11) that comprises the main liquid and the at least one main active product, said main reservoir (11) being connected to a main inlet (13) of a main conduit (14), and a first reservoir (15) that comprises the at least one surfactant, said first reservoir (15) being in fluid communication (16) with a first secondary inlet (17) of the main conduit (14), said main conduit (14) being connected to the main outlet (12) of the first module,
- or a main reservoir (21) that comprises the main liquid, said main reservoir (21) being connected to a main inlet (23) of a main conduit (24), a first reservoir (25) that comprises the at least one surfactant, said first reservoir (25) being in fluid communication (26) with a first secondary inlet (27) of the main conduit (24), and a second reservoir (28) that comprises the at least one main active product, said second reservoir (28) being in fluid communication (29) with a second secondary inlet (30) of the main conduit (24), said main conduit (24) being connected to the main outlet (22) of the first module,
- or a main inlet (33) of a main conduit (34) connected to an inlet of the main liquid, a first reservoir (35) that comprises the at least one surfactant and the at least one main active product, said first reservoir (35) being in fluid communication (36) with a first secondary inlet (37) of the main conduit (34), said main conduit (34) being connected to the main outlet (32) of the first module,
- or a main inlet (43) of a main conduit (44) connected to an inlet of the main liquid, a first reservoir (45) that comprises the at least one surfactant, said first reservoir (45) being in fluid communication (46) with a first secondary inlet (47) of the main conduit (44), and a second reservoir (48) that comprises the at least one active product, said second reservoir (48) being in fluid communication (49) with a second secondary inlet (50) of the main conduit (44), said main conduit (44) being connected to the main outlet (42) of the first module.

4. Equipment according to claim 1 or 2, **characterised in that** the second module further comprises means configured so that the foam produced by the second module comprises the at least one main active product and the first module comprises:
- either a single reservoir (51) that comprises the main liquid, the at least one surfactant and optionally at least one secondary active product, this reservoir (51) being connected to the main outlet (52) of the first module,
- or a main reservoir (61) that comprises the main liquid, and optionally at least one secondary active product, said main reservoir (61) being connected to a main inlet (63) of a main conduit (64), and a first reservoir (65) that comprises the at least one surfactant, said first reservoir (65) being in fluid communication (66) with a first secondary inlet (67) of the main conduit (64), said main conduit (64) being connected to the main outlet (62) of the first module,
- or a main reservoir (61) that comprises the main liquid, said main reservoir (61) being connected to a main inlet (63) of a main conduit (64), and a first reservoir (65) that comprises the at least one surfactant and optionally at least one secondary active product, said first reservoir (65) being in fluid communication (66) with a first secondary inlet (67) of the main conduit (64), said main conduit (64) being connected to the main outlet (62) of the first module,
- or a main reservoir (61) that comprises the main liquid and the at least one surfactant, said main reservoir (61) being connected to a main inlet (63) of a main conduit (64), and a first reservoir (65) that comprises at least one secondary active product, said first reservoir (65) being in fluid communication (66) with a first secondary inlet (67) of the main conduit (64), said main conduit (64) being connected to the main outlet (62) of the first module,
- or a main reservoir (71) that comprises the main liquid, said main reservoir (71) being connected to a main inlet (73) of a main conduit (74), a first reservoir (75) that comprises the at least one surfactant, said first reservoir (75) being in fluid communication (76) with a first secondary inlet (77) of the main conduit (74), and a second reservoir (78) that comprises at least one secondary active product, said second reservoir (78) being in fluid communication (79) with a second secondary inlet (80) of the main conduit (74), said main conduit (74) being connected to the main outlet (72) of the first module,
- or a main inlet (83) of a main conduit (84) connected to an inlet of the main liquid, a first reservoir (85) that comprises the at least one surfactant and optionally at least one secondary active product, said first reservoir (85) being in fluid communication (86) with a first secondary inlet (87) of the main conduit (84), said main conduit (84) being connected to the main outlet (82) of the first module,
- or a main inlet (93) of a main conduit (94) connected to an inlet of the main liquid, a first reservoir (95) that comprises the at least one surfactant, said first reservoir (95) being in fluid communication (96) with a first secondary inlet (97) of the main conduit (94), and a second reservoir (98) that comprises at least one secondary active product, said second reservoir (98) being in fluid communication (99) with a second secondary inlet (100) of the main conduit (94), said main conduit (94) being connected to the main outlet (92) of the first module.

5. Equipment according to any one of the preceding claims, **characterised in that** the second module comprises a main inlet (101, 111, 121) connected to the main outlet of the first module, said main inlet (101, 111, 121) being connected:
- either to at least one main nozzle (102), a grille (103) being arranged downstream of said at least one main nozzle (102), and, upstream of said at least one main nozzle (102), means configured to produce a blast (104) in the direction of the flow of the mixture present at the main inlet (101, 111, 121) of the second module,
- or to at least one main nozzle (112), a gas-inlet section (114) in the form of a casing being disposed downstream of said at least one main nozzle (112), the longitudinal axis of this inlet section (114) being parallel to or identical to the projection axis of said at least one main nozzle (112), and, downstream of this inlet section (114), a grid (113) disposed on the outlet of the inlet section (114),
- or to a final reservoir (122), said final reservoir comprising a bubbling system (123).

6. Equipment according to any one of claims 2 to 5, **characterised in that** the single reservoir of the first module or the main reservoir of the first module has an inlet connected to a pyrotechnic system, or to a compressed-gas storage and/or production system (214).

7. Equipment according to claim 6, **characterised in that** the compressed-gas storage and/or production system comprises a compressor and/or a pressurised gas cylinder.

8. Equipment according to any one of the preceding claims, **characterised in that** the first module comprises at least one third reservoir (140, 160, 201) that comprises the at least one additive, said third reservoir (140, 160, 201) being in fluid communication with a third secondary inlet (141, 161, 202) of the main conduit of the first module.

9. Equipment according to any one of the preceding claims, **characterised in that** the mixture delivered at the main outlet of the first module has an additive content varying from 0.0001 to 10% by weight, preferably from 0.001% to 1% by weight, with respect to the total weight of the mixture delivered at the main outlet of the first module.

10. Equipment according to any one of claims 1 to 2 and 4 to 9, **characterised in that** the means configured for the foam produced by the second module to comprise the at least one main active product, comprise an addition device that comprises a supplementary reservoir (180) that comprises the at least one main active product, the supplementary reservoir (180) being connected to at least one means able to add the at least one main active product to the mixture present at the main inlet of the second module.

11. Equipment according to claim 10, **characterised in that**, when the main inlet of the second module is connected to at least one main nozzle, the means able to add the at least one main active product to the mixture present at the main inlet of the second module comprises at least one supplementary nozzle (181), the flow of the at least one main active product being in the same direction as the flow of the mixture present at the main inlet of the second module.

12. Equipment according to any one of claims 4 to 11, **characterised in that** said at least one secondary active product is selected from dyes, fluorescent products, chemical and/or biological markers, irritant products, gases, and products destroying the muscular and/or thinking capabilities of a living being and radar reflectors.

13. Equipment according to any one of claims 1 to 12, **characterised in that** said at least one main active product and said at least one optional secondary active product are selected from irritant products and products destroying the muscular and/or thinking capabilities of a living being, and more particularly said at least one main active product and said at least one optional secondary active product are capsaicin, capsicum oleoresin or one of the derivatives thereof and nonivamide.

14. Motor vehicle, ship or aircraft comprising the equipment as defined in any one of claims 1 to 13.

15. Foam-production method comprising the following steps:
(i) a first module that delivers, at the main output thereof, a mixture comprising a main liquid and at least one surfactant, then
(ii) a second module connected to the main outlet of the first module that produces a foam that comprises the main liquid, the at least one surfactant and at least one main active product, the foam produced having a swell factor greater than 10, advantageously greater than 20, preferably varying from 20 to 2000, more preferentially varying from 40 to 2000, even more preferentially varying from 60 to 2000, even more preferentially varying from 150 to 2000, and in particular varying from 400 to 2000, **characterised in that** said at least one main active product is selected from irritant products and products destroying the muscular and/or thinking capabilities of a living being, the foam produced by the second module further comprising at least one viscosity-increasing additive, the viscosity-increasing additive being a gum arabic, the irritant product or products and the product or products destroying the muscular and/or thinking capabilities of a living being being encapsulated in the gum arabic.
